Europäisches Patentamt

European Patent Office

Office européen des brevets

(19)

(11) **EP 0 701 616 B1**

(12) **EUROPÄISCHE PATENTSCHRIFT**

(45) Veröffentlichungstag und Bekanntmachung des Hinweises auf die Patenterteilung:
**01.09.1999 Patentblatt 1999/35**

(21) Anmeldenummer: **94918392.5**

(22) Anmeldetag: **06.06.1994**

(51) Int Cl.⁶: **C12N 15/70**, C12N 15/63, C12N 15/12, C12P 21/02, A61K 38/18, C12N 1/21

(86) Internationale Anmeldenummer:
**PCT/EP94/01861**

(87) Internationale Veröffentlichungsnummer:
**WO 94/29462 (22.12.1994 Gazette 1994/28)**

(54) **MULTICISTRONISCHE EXPRESSION REKOMBINANTER GENE IN BAKTERIENZELLEN**

MULTICISTRONIC EXPRESSION OF RECOMBINANT GENES IN BACTERIAL CELLS

EXPRESSION MULTICISTRONIQUE DE GENES RECOMBINES DANS DES CELLULES BACTERIENNES

(84) Benannte Vertragsstaaten:
**DE ES FR GB IT NL**

(30) Priorität: **10.06.1993 DE 4319708**

(43) Veröffentlichungstag der Anmeldung:
**20.03.1996 Patentblatt 1996/12**

(73) Patentinhaber:
• **Beiersdorf Aktiengesellschaft**
 **D-20253 Hamburg (DE)**
• **Gesellschaft für biotechnologische Forschung mbH (GBF)**
 **38124 Braunschweig (DE)**

(72) Erfinder:
• **SCHNEPPE, Bernard**
 **D-38304 Wolfenbüttel (DE)**
• **McCARTHY, John, E., G.**
 **D-38124 Braunschweig (DE)**
• **EICHNER, Wolfram**
 **D-22301 Hamburg (DE)**

(74) Vertreter: **UEXKÜLL & STOLBERG**
 **Patentanwälte**
 **Beselerstrasse 4**
 **22607 Hamburg (DE)**

(56) Entgegenhaltungen:
**EP-A- 0 288 307      GB-A- 2 214 508**

• **EMBO JOURNAL Bd. 4, Nr. 2 , Februar 1985 Seiten 519 - 526 MCCARTHY, J.E.G. ET AL. 'Translational initiation frequency of atp genes from Escherichia coli: identification of an intercistronic sequence that enhances translation' in der Anmeldung erwähnt**
• **ADV. GENE TECHNOL. Bd. 2 , 1991 Seiten 145 - 175 MCCARTHY, J.E.G. 'Optimizing post-translational steps of gene expression in Escherichia coli' in der Anmeldung erwähnt**
• **TRENDS GENET. Bd. 6, Nr. 3 , 1990 Seiten 78 - 85 MCCARTHY, J.E.G. AND GUALERZI, C. 'Translational control of procaryotic expression' in der Anmeldung erwähnt**
• **DATABASE BIOSIS NR. 87081756 KRAVCHENKO, V.V. ET AL. 'Translation efficiency of distal gene polycistronic messenger RNA depends on mutual arrangement of regulatory signals of template' & BIOORG. KHIM. Bd. 14, Nr. 10 , 1988 Seiten 1372 - 1386 KRAVCHENKO, V.V. ET AL.**
• **GENE Bd. 143 , 1994 Seiten 201 - 209 SCHNEPPE, B. ET AL. 'Translational regulation of a recombinant operon containing human platelet-derived growth factor (PDGF)-encoding genes in Escherichia coli: genetic titration of the peptide chains of the heterodimer AB'**

**Beschreibung**

Die Erfindung betrifft die rekombinante Herstellung heteromerer Proteine in Bakterienzellen unter Verwendung multicistronischer Expressionsvektoren.

Eine wichtige allgemeine Strategie zur Erzeugung und Isolierung relativ großer Mengen eines rekombinanten Proteins wie beispielsweise eines Wachstumsfaktors ist die Überexpression der cDNA mit Hilfe entsprechender Expressionssysteme. Zur heterologen Genexpression werden verschiedene Systeme eingesetzt, u.a. Säugerzellen, das Baculovirussystem in Insektenzellen, Hefe, oder Bakterien wie *Bacillus subtilis* und *Escherichia coli.* Obwohl jedes dieser Systeme Vor- und Nachteile aufweist, wird weitaus am häufigsten mit dem Bakterium *E. coli* gearbeitet. Dieser Organismus bietet gute Voraussetzungen für die Entwicklung von Strategien zur Überproduktion fremder Gene, da er einerseits gentechnologisch leicht und ökonomisch günstig zu handhaben und andererseits im Vergleich zu allen anderen Systemen am umfassendsten erforscht ist. So existieren definierte Stämme - z.T. mit für die heterologe Genexpression günstigen Mutationen - und Vektorsysteme mit gut charakterisierten effektiven Steuerungselementen, die relativ einfach und schnell hohe Ausbeuten an rekombinantem Protein sowohl für akademische als auch für kommerzielle Zwecke ermöglichen.

Die Höhe der Ausbeute an rekombinantem Protein kann durch mehrere Faktoren gesteuert bzw. beeinflußt werden (Buell und Panayotatos, 1986; Glick und Whitney, 1987; Gold, 1990; McCarthy, 1991). Die in erster Linie Ausbeute-bestimmenden Kriterien für die heterologe Genexpression in *E. coli* sind jedoch die Effizienz der Transkription und der Translation eines Gens. Die Steuerung dieser Prozesse muß daher bei der Entwicklung bzw. Verwendung eines Expressionssystems beachtet werden. Um die effektive Transkription eines Gens zu gewährleisten, wird dieses im allgemeinen unter die Kontrolle eines "starken" Promotors gebracht. Damit die Fremdgenexpression nicht unnötig mit dem Metabolismus der Zelle interagiert und möglicherweise den Zellertrag oder die Plasmidstabilität herabsetzt, sollte der Promotor regulierbar, d. h. in einer bestimmten Wachstumsphase der Zellen aktivierbar sein. Die gebräuchlichsten Promotoren sind die des trp- oder lac-Operons von *E. coli,* der tac-Promotor, die Promotoren $P_L$ und $P_R$ des Bakteriophagen Lambda und der Promotor des Bakteriophagen T7 (Yansura und Henner, 1990; Yanisch-Perron et al., 1985; Stark, 1987; de Boer et al., 1983; Remaut et al., 1981; Schauder et al., 1987; Tabor und Richardson, 1985; Studier und Moffatt, 1986).

Die Translationsrate eines Gens wird in *E. coli* in erster Linie auf der Stufe der Initiation über die Translations-Initiations-Region (TIR) des Gens gesteuert. Dieser Bereich der mRNA ist funktionell definiert und legt sowohl den Ort als auch die Effizienz der Initiation der Translation fest. Er umfaßt neben der Shine/Dalgarno-Sequenz und einem hiervon im Abstand von 4-12 Basen gelegenen Startcodon auch weitere flankierende Bereiche der mRNA, die die Effizienz der Ribosomenbindung über ihre Primärsequenzen und Strukturen höherer Ordnung beeinflussen können (siehe unten). Während der 5' Bereich der TIR eines Gens einen nicht translatierten "leader" oder im Falle einer polycistronischen mRNA auch das Ende eines stromaufwärts gelegenen Cistrons beinhalten kann, kann der 3' Bereich bis in den zugehörigen kodierenden Genabschnitt selbst hineinreichen (McCarthy, 1991; McCarthy und Gualerzi, 1990; vergleiche Scherer et al., 1980).

Zur Gewährleistung der effektiven Translation eines rekombinanten Gens in *E. coli* existieren prinzipiell drei Strategien. Eine Möglichkeit besteht in der Expression des rekombinanten Gens als Teil eines Fusionsgens (Uhlen und Moks, 1990; Yansura, 1990). Dazu wird es unter Beibehaltung des Leserahmens stromabwärts der TIR und des 5' Bereiches eines gut exprimierbaren prokaryontischen Gens kloniert. Diese Konstruktion wird auch als translationelle Fusion bezeichnet. Da die TIR nicht verändert wird, kann in der Regel auch für das Fusionsgen von einer vergleichbar hohen Translationsrate ausgegangen werden. Mit Hilfe dieser Methode wurde zum ersten Mal gezeigt, daß Bakterien in der Lage sind, eukaryontische kodierende Sequenzen zu exprimieren (Villa-Komaroff et al., 1978; Seeburg et al., 1978; Martial et al., 1979). Sie ist von besonderer Bedeutung für die Herstellung von Antigenen und Vaccinen, wobei die Fusionsproteine direkt eingesetzt werden können (Kleid et al., 1981; Ellis et al., 1983; Van der Werf et al., 1983; Stanley und Luzio, 1984; Jacob et al., 1985; Cabradilla et al., 1986; Nilson und Abrahmsen, 1990), und für die stabile Synthese sehr kleiner Polypeptide oder Proteine in *E. coli,* da letztere als Teil eines Fusionsproteins vor proteolytischem Abbau durch bakterielle Proteasen geschützt werden können (Itakura et al., 1977; Shine et al., 1980; Ohsuye et al., 1983; Stanley und Luzio, 1984; Wingender et al., 1988; Yansura, 1990; Nilsson und Abrahmsen, 1990). Dabei spielt auch die in vielen Fällen beobachtete Aggregation der Fusionsproteine in Form von Einschlußkörpern eine Rolle (Marston et al., 1986; Kane and Hartley, 1988). Der N-terminale Proteinanteil kann außerdem so gewählt werden, daß die Aufreinigung des Fusionsproteins erleichtert wird (Ullman, 1984; Germino und Bastia, 1984; Brewer und Sassenfeld, 1985; Moks et al., 1987; Nilsson und Abrahmsen, 1990). Als Fusionsprotein im Cytoplasma exprimierte rekombinante Gene können bis zu 20% des gesamten Zellproteins ausmachen (Marston, 1986).

Der große Nachteil dieser Strategie ist jedoch, daß das nicht fusionierte rekombinante Protein erst nach der Entfernung des N-terminalen prokaryontischen Fusionsproteinanteils, der die biologische Aktivität des Proteins veringern kann und dessen antigene Eigenschaften verändert, erhalten werden kann. Die häufig zu diesem Zweck angewandte chemische Spaltung des Fusionsproteins mit dem Reagenz Cyanbromid, welches nach Methioninresten in der Proteinkette angreift, ist beschränkt auf Polypeptide, die keine internen Methioninreste tragen und säurestabil sind, da die Spaltung in 70% Ameisensäure durchgeführt wird (Itakura et al., 1977; Goeddel et al., 1979; Ohsuye et al., 1983; Peters et al., 1985; Marston, 1986; Hoppe et al., 1989, 1990). Eine Spaltung von Fusionsproteinen durch Säure- (Szoka et al., 1986; Wingender et al., 1989) oder Hydroxylaminbehandlung (Moks et al., 1987) zwischen den entsprechend eingebauten Aminosäuren Asparaginsäure und Prolin bzw. Asparagin und Glycin kann einerseits wiederum nur für rekombinante Genprodukte eingesetzt werden, die diese Kombination nicht aufweisen. Andererseits können bei diesen Methoden modifizierte Proteine entstehen, die gegenüber dem natürlich vorkommenden eine zusätzliche oder andere Aminosäure am N-terminalen Ende tragen (Szoka et al., 1986; Wingender et al., 1989) und im Falle der Säurebehandlung Asparaginreste, die die Amidgruppe verloren haben (Marston, 1986). Eine zusätzliche Aminosäure am N-Terminus oder deren Austausch gegen eine andere kann die Aktivität des rekombinanten Proteins beeinflussen (Potts et al., 1982; vergleiche Szoka et al., 1986) oder kann dessen antigene Eigenschaften derart verändern, daß es für eine möglicherweise geplante klinische Anwendung nicht geeignet ist. In anderen Fällen wurden zwischen prokaryontischem und rekombinantem Fusionsgenanteil Sequenzen eingebaut, die für spezifische Protease-Spaltstellen kodieren, sodaß durch enzymatische Spaltungen das natürliche rekombinante Protein wieder hergestellt werden konnte (Lee und Ullrich, 1984; Nagai und Thogersen, 1984; Germino und Bastia, 1984; Vardarajan et al., 1985; Nambiar et al., 1987; Brewer und Sassenfeld, 1985; Nilsson und Abrahmsen, 1990). Aber auch bei diesem Ansatz können Schwierigkeiten auftreten, wenn innerhalb des Fremdproteins protease-sensitive Bereiche enthalten sind (Shine et al., 1980; vergleiche Nambiar et al., 1987), das Fusionsprotein unter Bedingungen, die für die Aktivität der Proteasen notwendig sind, nicht löslich ist, oder die eingebaute Protease-Spaltstelle durch andere Proteinbereiche vor dem Protease-Angriff abgeschirmt wird (Marston, 1986; Wingender et al., 1989). Weiterhin wird die Bedeutung der Fusionsgenmethode für die Herstellung rekombinanter Proteine im großtechnischen Maßstab durch die Toxizität von Cyanbromid einerseits und die anfallenden Kosten für Proteasen andererseits gemindert.

Eine Abwandlung der oben angesprochenen Methode ist die Synthese rekombinanter Proteine fusioniert mit sogenannten bakteriellen Signalpeptidsequenzen (Marston, 1986; Nicaud et al., 1986; Stader und Silhavy, 1990). Diese Sequenzen vermitteln den Transport von Proteinen aus dem Cytoplasma in den periplasmatischen Raum von *E. coli* oder in selteneren Fällen auch in das Kulturmedium. Da das Signalpeptid aber während des Transports über die Cytoplasmamembran abgespalten wird, kann mit Hilfe dieser Methode bei entsprechend durchgeführter Fusion direkt das authentische rekombinante Protein erhalten werden. Die Lokalisation des Proteins im Periplasma von *E. coli* oder im Kulturmedium kann dessen anschließende Aufreinigung erleichtern und, obwohl auch Gegenteiliges bekannt ist (Gentz et al. 1988; Strauch und Beckwith, 1988), vor proteolytischem Abbau schützen (Talmadge und Gilbert, 1982; Swamy und Goldberg, 1982; Wong et al., 1988; Dalboge et al., 1989).

Mittlerweile gibt es eine Anzahl von Beispielen für die erfolgreiche Nutzung dieser Methode. Während die Sekretion rekombinanter Proteine ins Kulturmedium nur in wenigen Fällen gelang (Nagahari et al., 1985; Lord, 1985), wurde in der Mehrzahl der Arbeiten unter Verwendung verschiedener bakterieller Signalpeptidsequenzen der Export rekombinanter Proteine ins Periplasma von *E. coli* beschrieben (Talmadge et al., 1980; Zemel-Dreason und Zamir, 1984; Chang et al., 1987; Dalboge et al., 1989). Einige Arbeitsgruppen beobachteten passives Herauslecken des Proteins aus dem Periplasma (Wong et al., 1988; Obukowicz et al. 1988; Better et al., 1988, vergleiche Stader und Silhavy, 1990) oder erreichten dessen Freisetzung durch die Coexpression weiterer Gene, deren Produkte die äußere Membran von *E. coli* permeabel machten (Kato et al., 1987; Hsiung et al., 1989). In diesem Zusammenhang zeigt sich ein weiterer Vorteil des Exports rekombinanter Proteine in den periplasmatischen Raum von *E. coli.* Da in diesem Kompartiment stärker oxidierende Bedingungen herrschen als im Cytoplasma, können hier Proteine mit Disulfidbrükken solche Bindungen ausbilden und damit ihre korrekte Konformation erlangen (Chan et al., 1981; Pollitt und Zalkin, 1983; Emerick et al., 1985; Gray et al., 1985; Hsiung et al., 1986; Kato et al., 1987; Wong et al., 1988; Hsiung et al., 1989; Klein et al., 1991). In den zitierten Arbeiten wurden die Signalpeptidsequenzen jeweils korrekt prozessiert, die resultierenden rekombinanten Proteine waren löslich und, wenn untersucht, biologisch aktiv. Es wurden allerdings auch schon unlösliche Proteinaggregate im Periplasma gefunden (Takagi et al., 1988; Wong et al., 1988; Obukowicz et al., 1988). In Ausnahmefällen wurde auch für eukaryontische Proteine mit deren natürlichen Signalsequenzen die korrekte Prozessierung in *E. coli*-Zellen und der Transport in das Periplasma nachgewiesen (Talmadge et al., 1980; Zemel-Dreasen und Zamir, 1984; Gray et al., 1985).

Mit der Herstellung biologisch aktiver Antikörper-Fragmente wurde gezeigt, daß auch aus verschiedenen Untereinheiten bestehende rekombinante Proteine im Periplasma korrekt assembliert werden können (Better et al., 1988; Skerra und Plückthun, 1991; Sawyer und Blattner, 1991). Die weitere Dimerisierung von Fab- zu F(ab)$_2$-Fragmenten scheiterte jedoch (Carter et al., 1992).

Leider ließ sich bis heute aus den vorliegenden Ergebnissen keine universell einsetzbare Standardmethode bezüglich des Exports oder der Sekretion rekombinanter Proteine durch *E. coli* ableiten. Im Gegenteil scheint jedes Protein einen Spezialfall zu repräsentieren, da die Anwesenheit von Signalpeptiden zwar die Voraussetzung für den Transport über die Cytoplasmamembran ist, aber auch weitere Informationen aus den Sequenzbereichen des reifen Proteins dazu notwendig sind. So erwies sich in einigen Fällen die Synthese Signalpeptid tragender Proteine als toxisch für *E. coli,* weil die chimären Proteine in der Cytoplasmamembran stecken blieben und die Exportmaschinerie blockiert wurde (Bassford et al., 1979; Ito, 1981; Henning et al., 1983; Brosius, 1984). Weiterhin wurde erst in einem Fall der Export eines eukaryontischen cytoplasmatischen Proteins in das Periplasma von *E. coli* publiziert (Takahara et al., 1988). Aus diesen Ergebnissen kann man schließen, daß nicht jedes Protein exportierbar ist. Andere Arbeiten zeigten darüberhinaus, daß die Exportmaschinerie von *E. coli* nur eine begrenzte Kapazität besitzt, die lediglich eine moderate Syntheserate für das rekombinante Protein zuläßt (Obukowicz et al., 1988). Eine Steigerung der Rate hatte jeweils eine Anhäufung des unprozessierten Vorläuferproteins im Cytoplasma und eine Hemmung des Zellwachstums zur Folge (Hsiung et al., 1986; Hsiung et al., 1989; Takagi et al., 1988; Klein et al., 1991). Diese Tatsache bietet eine Erklärung für einen weiteren gravierenden Nachteil dieser Expressionsmethode, welcher darin begründet ist, daß bisher mit Ausnahme von Kato et al. (1987) (11,2 mg/Liter Kultur) im Vergleich zur cytoplasmatischen Expression nur sehr geringe Ausbeuten an rekombinantem Protein erzielt werden konnten (bis ca. 7% des gesamten Zellproteins bzw. ca 1-2 mg/Liter Kultur). Für kleine Proteine kann der Export ins Periplasma allerdings auch sehr effektiv sein (Dalboge et al., 1989). Der von Carter et al. (1992) publizierte Wert von 1-2 g Fab-Fragmenten pro Liter Kultur wurde allerdings nur durch ein spezielles Hochzelldichte-Fermentationsverfahren erreicht. Der Export von rekombinantem Protein ins Periplasma oder dessen Sekretion ins Kulturmedium wurde in neueren Expressionssystemen durch die Verwendung der N-terminalen *Staphylococcus* Protein A-Sequenz zwar deutlich effektiver gestaltet (Nilsson und Abrahsen, 1990). Bei den auf diese Weise erhaltenen Produkten handelt es sich jedoch jeweils wiederum um Fusionsproteine (vergleiche mit oben).

Die dritte prinzipielle Methode zur heterologen Genexpression in *E. coli,* die direkte cytoplasmatische Expression, zielt auf die Synthese nicht fusionierter ausschließlich durch das rekombinante Gen kodierter Proteine im Cytoplasma ab (McCarthy, 1991). Um das rekombinante Gen zu diesem Zweck mit einem effektiven bakteriellen Translationssteuerungssignal zu versehen, wird es im allgemeinen mit einem Startcodon in optimaler Entfernung an die nicht translatierte 5'-"leader"-Sequenz einer effektiven bakteriellen TIR angeschlossen. Derjenige Bereich einer TIR (s.o.), der nicht den codogenen 5'-Bereich des jeweiligen Cistrons einschließt, wird in dieser Anmeldung allgemein als Translations-Initiations Sequenz (TIS) bezeichnet. Diese Konstruktion, die auch als transkriptionelle Fusion bezeichnet wird, resultiert in einer neuen chimären TIR bestehend aus dem bakteriellen "leader" inklusive Shine/Dalgarno-Sequenz (TIS) und dem 5' Bereich des rekombinanten Gens. Die Notwendigkeit eines Startcodons am 5' Ende der rekombinanten Gensequenz für die Initiation der Translation hat bei dieser Methode den Nachteil, daß das Erstprodukt des Translationsprozesses jeweils einen Methioninrest als N-Terminus trägt. Dieser wird vielfach abgespalten, sodaß bei geeignetem Anschluß der Gensequenz an den bakteriellen "leader" das gewünschte authentische rekombinante Genprodukt erhalten wird (George et al., 1985; Winkler et al., 1985; Kronheim et al., 1986; Langley et al., 1987A; Tanizawa et al., 1987; Meyers et al., 1987). Demgegenüber steht eine Reihe von rekombinanten Genprodukten, bei denen diese Abspaltung nicht beobachtet wurde (Goeddel et al., 1979; Ikehara et al., 1984; Buell et al., 1984; Kaytes et al., 1986; Ivanoff et al., 1986; Miki et al., 1987; Denefle et al., 1987; Saito et al., 1987).

Ein weiterer Nachteil dieser Expressionsmethode kann im Angriff des Expressions-produktes durch cytoplasmatische Proteasen bestehen. Die Synthese Protease-sensitiver rekombinanter Genprodukte im Cytoplasma kann aber durch die Verwendung spezieller *E. coli*-Stämme, die Defekte im htpR-Gen oder in Proteasegenen tragen, erreicht werden (Boss et al., 1984; Buell et al., 1985; Mandecki et al., 1986). Außerdem wurde die Stabilisierung rekombinanter Proteine durch die Coexpression von Genen erreicht, die ein Produkt kodieren, das Fremdproteine schützt (Simon et al., 1983; Duvoisin et al., 1986) oder dieses in unlösliche Aggregate präzipitiert (Saito et al., 1987). In letzterem Fall wurde ein bicistronisches System bestehend aus zwei überlappenden Genen eingesetzt, das zu einem molaren Verhältnis der Genprodukte von 4,5 : 1 führte.

Beispiele für bekannte natürliche "leader"-Sequenzen bakterieller TIRs bzw. TISs, welche für die Initiation der Translation rekombinanter Gene in *E. coli* verwendet wurden, wird die des lacZ-Gens aus *E. coli* (Taniguchi et al., 1980; de Boer et al., 1983), des Replicase-Gens des RNA-Phagen MS2 (Remaut et al., 1983; Sollazo et al., 1985), des lpp-Gens aus *E. coli* (Nakamura und Inouye, 1982), des atpE-Gens aus *E. coli* (McCarthy et al., 1985; Schauder et al., 1987; Belev et al., 1991), des cro-Gens des Phagen Lambda (Sollazzo et al., 1985) und des Gens10 des Phagen T7 (Studier et al., 1990; Olins und Rangwala, 1990). Ferner wurden synthetische "leader" verwendet (Jay et al., 1981; Crowl et al., 1985; Stanssens et al., 1985). Die chimäre TIR sollte eine relativ offene Konformation aufweisen, um die effektive Bindung von Ribosomen zu erlauben. Sollte die TIR jedoch in Sekundärstrukturen der mRNA eingebettet sein, so kann durch in vitro-Mutagenese oder durch die Verwendung anderer, auch in vitro synthetisierter oder modifizierter, bakterieller "leader"-Sequenzen deren Stabilität verringert und die Genexpression damit deutlich gesteigert werden (Buell et al., 1985; Stanssens et al., 1985; Lee et al., 1987; Schauder und McCarthy, 1989; Spanjaard et al., 1989; Gross et al., 1990; McCarthy, 1991). Zur Vereinfachung solcher Optimierungen existieren besondere Expressionsvektoren, die auch als Einzelstrang-DNA zur Durchführung der in vitro-Mutagenese isoliert werden können (Solazzo et al., 1985; Belev et al., 1991) oder in denen bakterielle "leader" als Cassetten ausgetauscht werden können (Schauder et al., 1987; Belev et al., 1991). In einer anderen Strategie wurde die Aktivierung durch Strukturen höherer Ordnung maskierter TIRs rekombinanter Gene über translationelle Kopplung erreicht (Schoner et al., 1984; Spanjaard et al.,

1989). Die Sekundärstrukturen der TIRs wurden dabei durch elongierende Ribosomen geöffnet, die entsprechend stromaufwärts der rekombinanten Gene plazierte kurze Präcistrons translatieren.

Direkt exprimierte rekombinante Genprodukte können bis zu ca. 20-30% des gesamten Zellproteins von *E. coli* ausmachen. In vielen Fällen wurde die Aggregation der Proteine in Form von Einschlußkörpern im Cytoplasma beobachtet (s. Übersichtsartikel von Marston, 1986; Kane und Hartley, 1988). Die Bildung dieser unlöslichen Aggregate kann aus mehreren Gründen vorteilhaft sein. Die Isolierung der Einschlußkörper mit dem rekombinanten Protein ist einfach und resultiert bereits in einem Produkt von mehr als 50% Reinheit. Das ermöglicht eine Zeit und Kosten sparende Aufreinigungsprozedur, da im allgemeinen durch lediglich einen weiteren Schritt zu mehr als 90% reine Proteinpräparationen gewonnen werden können. Weiterhin bietet die Aggregation von Fremdprotein in *E. coli*-Zellen Schutz vor Proteasen (Saito et al., 1987), was den Vorteil homogener Proteinpräparationen bietet, da proteolytische Spaltprodukte oft schwierig zu entfernen sind. Das Problem ist jedoch, die unlöslichen und biologisch inaktiven Proteine wieder zu solubilisieren und in ihre native und aktive Form zu überführen. Die erfolgreiche Renaturierung ist allerdings mittlerweile für eine Vielzahl rekombinanter Proteine beschrieben (Boss et al., 1984; Cabilly et al., 1984; Marston et al., 1984; Simons et al., 1984; Gill et al., 1985; George et al., 1985; Winkler et al., 1985; Zurawski et al., 1986; Kronheim et al., 1986; Tsuje et al., 1987; Langley et al., 1987B; Denefle et al., 1987).

Andere rekombinante Proteine ließen sich im Cytoplasma in löslicher Form mit hoher Rate direkt exprimieren (Pennica et al., 1985; Gross et al., 1985; Kishimoto et al., 1986; Meyers et al., 1987; Tanizawa et al., 1987; Levi et al., 1987). Die Produkte waren zwar biologisch aktiv, jedoch war meistens eine aufwendige Prozedur zu deren Aufreinigung erforderlich. Der Anteil an löslichem rekombinantem Protein wird stark durch die Inkubationstemperatur der rekombinanten Zellen beeinflußt (Schein, 1989).

In der Regel wurden bisher im Cytoplasma von <u>*E. coli*</u> oder anderen Bakterien rekombinante Proteine stets einzeln exprimiert (vergleiche oben). Aber insbesondere in Fällen, in denen eine *in vitro* Dimerisierung verschiedener Proteinketten oder der Einbau verschiedener Proteinuntereinheiten in Komplexe angestrebt wird, wären auch Systeme wünschenswert, die die Coexpression von Proteinen in einer Zelle erlauben.

Für eine Multimerisierung von Proteinen, die die Ausbildung von Disulfidbrücken erfordert, **direkt** im bakteriellen Cytoplasma erweisen sich allerdings die dort herrschenden reduzierenden Bedingungen (Kane und Hartley, 1988; Tuggle und Fuchs, 1985; vergleiche oben) oftmals als großes Problem. Dieses wird besonders deutlich bei der Synthese von Antikörperfragmenten in *E. coli*. Während bei der cytoplasmatischen Coexpression von Genen für schwere und leichte Antikörperketten keine aktiven d.h. korrekt assemblierten Antikörperfragmente erhalten wurden (Cabilly et al., 1984; Boss et al., 1984), konnte durch die Sekretion der Ketten ins Periplasma das Problem gelöst und funktionelle Produkte erzielt werden (Better et al., 1988; Skerra und Plückthun, 1991; Sawyer und Blattner, 1991). In letzteren Arbeiten wurden die Gene der Antikörper-Domänen mit Sequenzen, die für bakterielle Signalpeptide kodieren, fusioniert und in Form einer bicistronischen mRNA von einem gemeinsamen Promotor aus transkribiert. Die Fusion von zwei rekombinanten Genen mit den gleichen Signalsequenzen inklusive Ribosomen-Bindestelle führte zu identischen und nichtmodifizierten bakteriellen TIR-Sequenzen zur Steuerung der Translationsrate beider Gene (Better et al., 1988; Sawyer und Blattner, 1991; Carter et al., 1992). Das stöchiometrische Verhältnis der Genprodukte wurde in keinem Falle überprüft oder gar versucht, dieses über die Variation der TIR- bzw. TIS-Sequenzen auf der Stufe der Initiation der Translation zu beeinflussen. Im Falle der cytoplasmatischen Expression von Immunglobulinfragmenten wurde die Coexpression mit Hilfe zweier verschiedener Plasmide erreicht (Cabilly et al., 1984; Boss et al., 1984). Bei dieser Methode ist es nicht möglich - vielleicht auch nicht gewünscht - , die Syntheseraten der rekombinanten Proteine aufeinander abzustimmen, und es wurden daher unterschiedliche Ausbeuten an schweren und leichten Antikörperketten erhalten. Dabei spielte auch differentielle Plasmidstabilität eine Rolle (Boss et al., 1984).

Zusammenfassend kann festgestellt werden, daß die gebräuchlichen und hier diskutierten Strategien zur heterologen Genexpression in *E. coli* letztlich zwei übergeordneten grundsätzlichen Kategorien zuzuordnen sind. In einem Fall wird durch die Sekretion der Proteinuntereinheiten in das Periplasma versucht, biologisch aktives Protein zu erhalten. Da diese Strategie nicht universell anwendbar ist und zumeist nur limitierte Produktausbeuten zuläßt, wird daher für die Produktion von Proteinen im biotechnologischen Maßstab in der Regel die cytoplasmatische Expression bevorzugt. Für die Gewinnung von Heteromeren wurden bisher entweder verschiedene *E. coli*-Stämme oder verschiedene Plasmide in einem Stamm verwendet. Die multicistronische Expression signalpeptidfreier, für Fremdproteine kodierender Gene in Bakterienzellen ist bisher im Stand der Technik nicht bekannt. Insbesondere ist es nicht möglich, die Expressionsrate der jeweiligen Komponenten der Heteromere zu steuern.

Eine derartige Steuerung der Syntheseraten cytoplasmatisch coexprimierter Untereinheiten heteromerer Proteine ist jedoch von großem wirtschaftlichen Interesse. So ist es sinnvoll, die Syntheserate den stöchiometrischen Verhältnissen gemäß einzustellen, die für eine nachfolgende *in vitro* Dimerisierung (also beispielsweise 1:1) oder den Zusammenbau zu einem Multi-Untereinheiten-Proteinkomplex günstig oder notwendig sind, wenn die Proteinmonomere unter Schaffung oder Beibehaltung dieses Verhältnisses in nativer oder denaturierter Form zusammen isoliert und/oder zum Multimer rekonstituiert werden können. Unterschiede im Löslichkeitsverhalten oder selektive Ausbeuteverluste, die für die von den verschiedenen Cistrons codierten Proteinuntereinheiten im Verlauf des jeweiligen Proteinreinigungsverfahrens auftreten, können maßgeblichen Einfluß auf die Einstellung der relativen Syntheseraten haben. So ist es beispielsweise von Vorteil, ein für die Heterodimerisierung scheinbar optimales stöchiometrisches Expressionsverhältnis der Untereinheiten von 1:1 dahingehend zu verändern, daß die Proteinuntereinheit mit geringerer Löslichkeit oder den höchsten relativen Ausbeuteverlusten bei der Reinigung durch Hochregulation der Translationseffizienz am korrespondierenden Cistron in der Menge den jeweiligen Anforderungen angepaßt wird. Umgekehrt kommt auch eine negative Regulation des besser exprimierten Cistrons in Betracht.

Die Steuerung der Syntheseraten ist für die bereits diskutierten Antikörper-Komponenten sowie unter anderem für heteromere Proteine wie VEGF, Scatter-Faktor (HGF-SF), Mitglieder der TGF-β Superfamilie, Bone Morphogenic Protein (BMP), Faktoren der Integrin/Cadherin Familie, Histokompatibilitätsantigene, Hämoglobin, T-Zell Rezeptoren oder das AB-Heterodimer des Wachstumsfaktors aus Blutplättchen (PDGF) bzw. deren natürliche oder synthetische Varianten interessant.

Der Wachstumsfaktor aus Blutplättchen (Platelet-Derived Growth Factor, PDGF) ist ein hochaktives Mitogen für Zellen mesenchymalen Ursprungs sowie glattmuskuläre Zellen (Kohler und Lipton, 1974; Ross et al., 1974). Schon sehr frühzeitig wurde vermutet, daß PDGF *in vivo* maßgeblich an lokalen Wundheilungsprozessen beteiligt ist (Ross et al. 1974; Deuel & Huang, 1984). Die Hypothese wird durch aktuelle klinische Befunde (Mustoe et al., 1989; Pierce et al., 1989; Greenhalgh et al., 1990, Pierce et al., 1991A, 1991B; Robson et al., 1992) erhärtet.

Aus menschlichen Thrombozyten aufgereinigtes PDGF besteht aus zwei unterschiedlichen, aber nahe verwandten Polypeptidketten, die durch Disulfidbrücken miteinander verknüpft sind. Unter reduzierenden Bedingungen zerfällt das dimere PDGF in seine monomeren, biologisch inaktiven Untereinheiten, wovon die größere ($M_r$ 15-17.000 D) als PDGF-A-Kette und die kleinere ($M_r$ 14.000 D) als PDGF-B-Kette bezeichnet wurde (Johnsson et al., 1984).

Die Proteinketten PDGF-A und -B werden von verschiedenen Genen kodiert. Die vollständige Struktur beider Genprodukte konnte durch cDNA-Klonierung aufgeklärt werden (Ratner et al., 1985, Betsholtz et al., 1986). Dabei ergab sich, daß beide PDGF-Moleküle zunächst als ungewöhnlich lange Vorläufermoleküle, sog. precursoren, synthetisiert und anschließend intrazellulär zu den reifen PDGF-Ketten prozessiert werden. Durch alternatives Splicen lassen sich zwei verschiedene PDGF-A-Transkripte erklären, die sich durch An- oder Abwesenheit eines 69-bp Segmentes im 3'-Bereich erklären (Betsholtz et al., 1986; Wise et al., 1989). Durch dieses Insert kommt es zu einer Änderung im codierenden Abschnitt mit der Folge, daß eine kurze (PDGF-$A_K$, 110 Aminosäuren) und eine lange (PDGF-$A_L$, 125 Aminosäuren) Form der PDGF-A-Kette gebildet wird. Beide Varianten sind als normale zelluläre Proteine nebeneinander nachweisbar, wobei die kürzere Form die häufigere Spezies ist (Matoskova et al., 1989; Young et al., 1990). Die höhere Affinität zum PDGF-α-Rezeptor könnte der Grund für die stärkere mitogene Potenz von PDGF-$A_K$ gegenüber PDGF-$A_L$ sein.

Beide Gene sind auf unterschiedlichen Chromosomen lokalisiert und zeigen einen hohen Homologiegrad. Eine Vielzahl von Arbeiten zeigt, daß beide Gene unterschiedlichen Regulationsmechanismen unterliegen. Eine Folge davon ist, daß beide PDGF-Ketten von verschiedenen Zelltypen normalerweise in unterschiedlichen Verhältnissen zueinander produziert werden.

Alle drei möglichen Isoformen des PDGF (AA, AB und BB) kommen natürlicherweise vor und sind in Thrombozyten in sog. α-Granula gespeichert. Aus überlagerten menschlichen Blutplättchen kann neben dem die Hauptmenge ausmachenden PDGF-AB Heterodimer auch PDGF-BB zu etwa 30% isoliert werden (Hammacher et al., 1988).

Frisch präparierte Blutplättchen enthalten auch einen hohen Anteil (27%) an PDGF-AA (Hart et al., 1990). Es kann daher angenommen werden, daß in den Vorläuferzellen der Thrombozyten, den Megakaryozyten, die PDGF-Isoformen AA, AB und BB im statistischen 1:2:1-Verhältnis zueinander hergestellt werden und daß daher der Anteil beider Homodimere etwa dem AB-Heterodimer-Anteil entspricht. Die Konzentration jeder PDGF-Spezies im Thrombozyten sollte direkt korrelieren mit ihrer individuellen Bedeutung im Wundheilungsgeschehen, wodurch insbesondere das PDGF-AB eine herausragende Rolle auf der Suche nach einem "Wundheilungshormon" bekommt.

Die geringen Ausbeuten bekannter Reinigungsprotokolle sind ein Grund dafür, daß es außerordentlich problematisch ist, PDGF in nenenswerten Mengen aus natürlichen Quellen zu isolieren. Noch weit weniger aussichtsreich ist es zu versuchen, die im humanen Thrombozyten-PDGF enthalten Isoformen (PDGF-AA, -AB, -BB; Hammacher et al., 1988) getrennt zu isolieren. Alle verschiedenen Isoformen besitzen biologische Aktivität *in vitro.* Erst die Verfügbarkeit der hochreinen, rekombinanten PDGF-Isoformen (Hoppe et al., 1989; Hoppe et al., 1990) machte vergleichende Studien zur Differenzierung der unterschiedlichen Wirkungsspektren der verschiedenen PDGF-Spezies möglich. Mittlerweile belegen eine Reihe von Untersuchungen die unterschiedliche Potenz von PDGF-AA, AB und BB im Chemotaxis- und DNA-Proliferationstest (Hosang et al., 1989; Nister et al., 1988; Reilly & Broski, 1989; Siegbahn et al., 1990), sowie deren unterschiedlichen Einfluß auf die Freisetzung von Inositol 1,4,5-Trisphosphat, Produktion von Diacylglycerol und $[Ca^{2+}]_i$-Mobilisierung (Block et al., 1989; Sachinidis et al., 1990A; Sachinidis et al., 1990B). Zwei unterschiedliche PDGF-Rezeptorpopulationen, von denen der PDGF-α-Rezeptor alle PDGF-Isoformen und der β-Rezeptor nur PDGF-BB bindet (Hart et al., 1988; Heldin et al., 1988) liefern eine plausible Erklärung dafür, wie sich Wirkungsunterschiede der PDGF-Isoformen über deren unterschiedliche Potenz zur Rezeptoraktivierung entfalten können. Die meßbaren unterschiedlichen *in vitro*-Effekte der PDGF-Isoformen, zusammen mit dem Nachweis zweier verschiedenener Rezeptorpopulationen, lassen Rückschlüsse auf unterschiedliche *in vivo* Wirkungsspektren von PDGF-AA, AB und BB zu. Daher ist die Produktion von reinem PDGF-AB, ohne PDGF-BB oder PDGF-AA als Begleitproteine, wünschenswert.

Für die Expression von PDGF oder PDGF-Analogen wurden zahlreiche der eingangs erwähnten Expressionssysteme (Hefe, Bakterien, Säugerzellen) eingesetzt. Versuche, die PDGF-Ketten A und B in heterologen Systemen zu exprimieren, haben sich bisher im wesentlichen auf die nachfolgend genannten Beispiele beschränkt.

Erstmals gelang Kelly et al (1985) die Expression von biologisch aktiven PDGF-BB Homodimeren in Hefezellen. Ein wesentlicher Nachteil der bisher publizierten Strategien zur Herstellung von heterodimerem PDGF-AB oder PDGF-AB Analogen in eukaryontischen Zellen, beispielsweise rekombinanten CHO-Zellen (Östman et al., 1988) oder mit Hilfe von Hefe-Expressionssystemen [EP 0 259 632] ist die nicht beeinflußbare Coexpression unerwünschter homodimerer PDGF-Nebenprodukte, die in nachgeschalteten Protein-Reinigungsprozessen entfernt werden müssen. Die Quantifizierung der in CHO-Zellen exprimierten unterschiedlichen PDGF-Dimere ergab 19% für PDGF-AA, 69% für PDGF-AB und 12% für PDGF-BB (Östman et al., 1988).

Erste Versuche, die PDGF-B Kette in Prokaryonten zu exprimieren, führten nicht zu biologisch aktiven Produkten (Devare et al. 1984; Wang & Williams, 1984), da in diesen Zellen das für die spontane Assoziation der monomeren Untereinheiten notwendige Milieu fehlt. Eine erfolgreiche *in* vitro-Renaturierung von PDGF-BB Homodimeren wurde erstmals von Hoppe et al (1989, s.a. WO 90/04035) beschrieben. Nach Expression der maturen PDGF-B Kette als Fusionsprotein in *E. coli* und anschließender Freisetzung des monomeren PDGF-B durch chemische Spaltung gelang die Rekonstitution der Monomere zu einem wachstumsstimulierenden rPDGF-BB. In gleicher Weise wurde auch wachstumsstimulierendes PDGF-AA und AB gewonnen (Hoppe et al., 1990, s.a. WO 90/08163). Als Folge der Fusionsprotein-Expression weist die nach diesem Verfahren isolierte PDGF-B Kette eine N-terminale Verkürzung um 12 Aminosäuren, sowie eine C-Terminale Verlängerung um 5 Aminosäuren auf. In WO 91/08762 wird ein weitgegend analoges Verfahren für die Herstellung von PDGF-AB in Prokaryonten beschrieben. Beide PDGF-Ketten wurden hier durch direkte cytoplasmatische Expression gewonnen.

Weder existierte bisher ein Hochleistungsexpressionssytem zur direkten cytoplasmatischen Expression der PDGF-Ketten in Bakterien noch wurde ein bicistronisches System zur gemeinsamen Expression beider Ketten in derartigen Zellen beschrieben.

Aufgabe der Erfindung ist es daher, Verfahren zur rekombinanten Herstellung multimerer Proteine in Bakterienzellen und Mittel zur Verwendung in diesen Verfahren zu schaffen, mit deren Hilfe es möglich ist, die Komponenten und/oder Untereinheiten multimerer Proteine in ein und derselben Bakterienzelle zu exprimieren und gleichzeitig die Syntheseraten der cytoplasmatisch koexprimierten Proteinuntereinheiten entsprechend den stöchiometrischen Verhältnissen zu steuern, die für eine nachfolgende *in vitro* Multimerisierung oder den Zusammenbau zu einem heteromeren Proteinkomplex wünschenswert oder notwendig sind. Insbesondere sollen Verfahren und Mittel geschaffen werden, mit Hilfe derer die Produktion rekombinanter Proteine in großen Mengen zu wirtschaftlichen Bedingungen möglich ist.

Zur Lösung der Aufgabe wird erfindungsgemäß ein Verfahren zur rekombinanten Herstellung multimerer Proteine in Bakterienzellen vorgeschlagen, welches dadurch gekennzeichnet ist, daß man

a) die Zellen mit einem Vektor transformiert, der eine multicistronische Expressionseinheit der allgemeinen Formel

$$p - (TIS - C)_n$$

für die simultane und direkte Expression der Komponenten und/oder Untereinheiten eines multimeren Proteins in ein und derselben Bakterienzelle enthält, in der

"p" ein bakterieller transkriptionaler Promotor und

"$(TIS - C)_n$" repetitive Gruppen sind, innerhalb derer

"C" jeweils für eine Untereinheit oder eine Komponente eines multimeren Proteins kodierende DNA-Sequenzen sind, die keine für ein Signalpeptid kodierende Region umfassen, wobei die Sequenzen "C" der aufeinander folgenden Gruppen "TIS - C" untereinander gleich und/oder verschieden sein können,

"TIS" synthetische und/oder natürliche bakterielle nicht translatierte "leader" Sequenzen sind, die jeweils mit "C" eine chimäre "TIR" bilden, wobei die Sequenzen "TIS" der aufeinander folgenden Gruppen "TIS - C" untereinander gleich und/ oder verschieden sein können, und

n eine natürliche Zahl ≥ 2 ist,

wobei "p", "TIS" und "C" jeweils operativ miteinander verknüpft sind und die Expressionsrate der Protein-kodierenden Sequenzen "C" durch Auswahl der jeweils zugehörigen Sequenz "TIS" den stöchiometrischen Verhältnissen gemäß eingestellt wird, die für die nachfolgende Renaturierung und/oder Rekonstitution notwendig ist,

b) die transformierten Zellen in einem geeigneten Nährmedium kultiviert und anschließend

c) die Zellen von dem Medium abtrennt, die koexprimierten Expressionsprodukte der Sequenzen "C" aus dem Cytoplasma der Zellen isoliert, gegebenenfalls renaturiert und zu dem funktionellen multimeren Protein rekonstituiert.

In dem Verfahren gemäß der vorliegenden Erfindung werden die rekombinanten Gene in Form einer polycistronischen mRNA von einem gemeinsamen Promotor aus transkribiert. Die gewünschten definierten Syntheseraten der Genprodukte bzw. ihr stöchiometrisches Verhältnis zueinander, das für die weiteren Renaturierungs- und Rekonstitutionsexperimente notwendig ist, wird mittels "genetischer Titration" auf der Ebene der Initiation der Translation eingestellt. Zu diesem Zweck werden für jedes der cotranskribierten Cistrons chimäre TIRs bestehend aus bakteriellen oder synthetischen nicht-translatierten DNA-Sequenzen "TIS" einerseits und den angrenzenden rekombinanten Nukleotidsequenzen der kodierenden Gene andererseits so ausgewählt bzw. konstruiert oder modifiziert, daß die gewünschte Translationseffizienz erreicht wird. Dies kann dadurch erreicht werden, daß für das erste Cistron der multicistronischen Expressionskassette gemäß Erfindung eine effiziente natürlicherweise vorkommende TIS wie z. B. die des lacZ-Gens aus *E. coli* (Taniguchi et al., 1980; de Boer et al., 1983), des Replicase-Gens des RNA-Phagen MS2 (Remaut et al., 1983; Sollazo et al., 1985), des lpp-Gens aus *E. coli* (Nakamura und Inouye, 1982), des atpE-Gens aus *E. coli* (McCarthy et al., 1985; Schauder et al., 1987; Belev et al., 1991), des cro-Gens des Phagen Lambda (Sollazzo et al., 1985) und des Gens10 des Phagen T7 (Studier et al., 1990; Olins und Rangwala, 1990) oder ein synthetischer "leader" [vergleiche Jay et al. (1981), Crowl et al. (1985), Stanssens et al. (1985) und andere] eingesetzt wird und über Auswahl und ggf. Modifikation der TIS des zweiten und aller weiteren Cistrons die Expressionshöhe der nachfolgenden Cistrons auf den gewünschten Wert titriert wird. Als für diese Zwecke besonders geeignet erwies sich die erfindungsgemäß beschriebene TIS B-Sequenz (SEQ ID NO: 15). Durch eine Modifikation der TIS-Sequenzen bzw. der Effizienzen der Translations-Initiation der Gene einer Transkriptionseinheit lassen sich auch andere Faktoren, die die Effizienz der Translation eines der Gene beeinflussen können, wie z. B. differentielle Stabilitäten in mRNA-Abschnitten der verschiedenen Cistrons kontrolliert kompensieren, ohne daß in die kodierenden Bereiche der rekombinanten DNA-Sequenzen eingegriffen werden muß. Wenn beispielsweise für beide Cistrons einer bicistronischen Expressionskassette gleiche TIS-Sequenzen eingesetzt werden, ist in der Regel eine 1:1 Stöchiometrie der Genexpression nicht zu erwarten. Wie bereits dargelegt, beinhaltet nämlich eine TIR in ihrer Gesamtheit neben der TIS auch den extremen 5'-Bereich der codierenden Sequenz des nachfolgenden Cistrons. Da sich die codogenen Sequenzen in einem bi- oder multicistronischen Konstrukt üblicherweise unterscheiden, liegen selbst mit gleichen TIS-Sequenzen ungleiche TIRs vor mit der Folge, daß die Translationseffizienzen in der Regel unterschiedlich sein werden. Neben der Insertion verschiedener TIS-Sequenzen vor das jeweils stromabwärts lokalisierte Cistron und nachfolgender Ermittlung des Expressionsergebnisses ist es auch möglich, eine (Fein-) Regulation der Expressionshöhe nach Sekundärstrukturanalyse der abgeleiteten mRNA durchzuführen. Dabei wird die mRNA-Struktur im Bereich der TIR durch gebräuchliche Computerprogramme, z. B. nach Zuker und Stiegler (1981), errechnet und dann im Hinblick auf Sekundärstrukturen, die die Translations-Initiation stören, analysiert. In der Regel kann durch die Eliminierung von Basenpaarungen insbesondere zwischen G-C (dreifache Wasserstoffbrückenbindung) im Bereich der TIR durch eine Destabilisierung von Strukturen höherer Ordnung eine verbesserte Zugänglichkeit von Ribosomen an die Ribosomenbindestelle und damit eine Erhöhung der Effizienz der Initiation der Translation erreicht werden. Die Einführung von z. B. zusätzlichen G-C Basenpaaren in den gleichen Bereich der TIR hat einen gegenteiligen Effekt, vgl. McCarthy (1991).

Die Erfindung schließt ferner Mittel zur Durchführung des Verfahrens ein, insbesondere multicistronische Expressionseinheiten zur Verwendung in dem Verfahren nach Anspruch 1, welche durch die allgemeine Formel

$$p - (TIS - C)_n$$

gekennzeichnet sind, in denen "p", "TIS" und "C" die oben angegebene Bedeutung haben.

Erfindungsgemäß ist "n" vorzugsweise eine natürliche Zahl von 2 bis 10, und in besonders bevorzugter Weise 2 oder 3.

Als Promotoren "p" kommen alle diejenigen Promotoren in Betracht, die in prokaryontischen Zellen wirksam sind, d. h., die die Genexpression in Prokaryonten initiieren können. Insbesondere können alle konstitutiven und induzierbaren Promotoren, beispielsweise die des <u>trp</u>- oder <u>lac</u>-Operons von *E. coli,* der tac-Promotor, die Promotoren $P_L$ und $P_R$ des Bakteriophagen Lambda, der Promotor des Bakteriophagen T7 etc. verwendet werden. Erfindungsgemäß ist der $P_R$ Promotor des Bakteriophagen Lambda bevorzugt.

Die multicistronischen Expressionseinheiten zur Verwendung im Rahmen der Erfindung können an ihrem 3'-Ende operativ mit einem bakteriellen Transkriptions-Terminator verknüpft sein. Ferner kann die Expressionseinheit mit einem Gen "r" mit eigenem Promotor gekoppelt sein, welches für ein Repressormolekül für den Promotor "p" kodiert.

Gemäß einer besonders bevorzugten Ausführungsform der Erfindung ist "p" der $\lambda P_R$-Promotor oder der $\lambda P_L$-Promotor und "r" das cI857-Gen für den temperatursensitiven $\lambda$-Repressor.

Wie oben im einzelnen dargelegt, können die erfindungsgemäß verwendeten "TIS"-Sequenzen natürlichen Ursprungs sein oder es kann sich um modifizierte natürliche oder synthetische Sequenzen handeln. Erfindungsgemäß besonders bevorzugt sind die "TIS"-Sequenzen TIS B gemäß SEQ. ID. NO: 15 und TIS E gemäß SEQ. ID. NO: 14.

Die Sequenzen "C" können rekombinante DNA-Sequenzen sein, die für Untereinheiten heteromerer Proteine wie PDGF-AB, VEGF, Scatter-Factor (HGF-SF), Mitglieder der TGF-$\beta$ Superfamilie, Bone Morphogenic Protein (BMP), Faktoren der Integrin/Cadherin Familie, Immunglobuline, Histokompatibilitätsantigene, Hämoglobin oder T-Zell Rezeptoren bzw. deren natürliche oder synthetische Varianten kodieren.

Gemäß einer besonders bevorzugten Ausführungsform der Erfindung sind in der verwendeten multicistronischen Expressioneinheit "n" gleich 2 und die Sequenzen "C" der beiden repetitiven Einheiten "(TIS - C)$_2$" enthalten alternativ ein für die A- oder die B-Kette von PDGF oder ein biologisch aktives Analogon oder ein Fragment derselben kodierendes Gen, wobei beide Sequenzen gleichzeitig in der Expressionseinheit vertreten sind. Gemäß einer besonders bevorzugten Ausführungsform der Erfindung handelt es sich bei einer der Sequenzen "C" um die PDGF-$A_K$-Sequenz (SEQ. ID. NO: 12) oder die PDGF-$A_L$-Sequenz, welche für die reife PDGF-A-Kette kodiert. In einem derartigen Konstrukt ist vorzugsweise die jeweils andere "C"-Sequenz die vollständige PDGF-B-Sequenz gemäß SEQ. ID. NO: 10, welche für die reife PDGF-B-Kette SEQ. ID. NO: 11 kodiert oder das v-sis-Gen aus Simian Sarcoma Virus oder Varianten dieser Sequenzen.

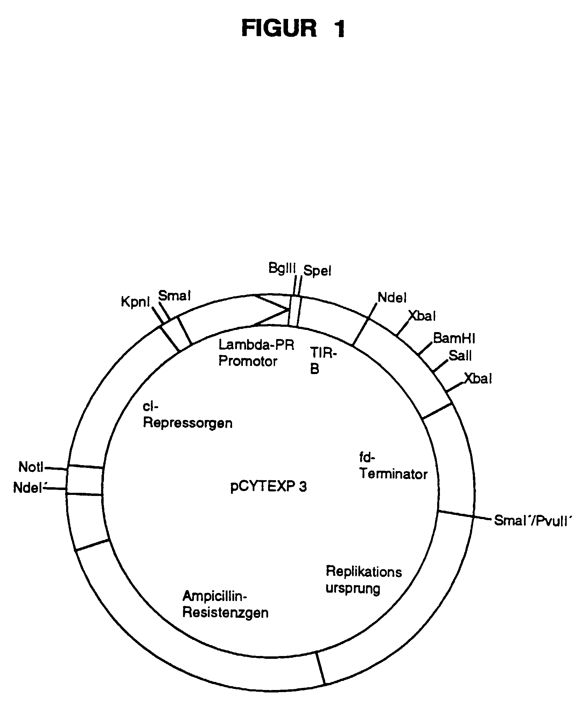

Die Erfindung schließt ferner rekombinante DNA-Vektoren ein, welche die erfindungsgemäßen multicistronischen Expressionseinheiten enthalten und in dem erfindungsgemäßen Verfahren zum Transformieren der Bakterienzelle verwendet werden. Ein erfindungsgemäß besonders bevorzugter Vektor ist in Figur 1 und dessen Herstellung in Abschnitt 2 Punkt 1 dargestellt. Schließlich betrifft die Erfindung prokaryontische Wirtszellen, die mit den zuvor genannten Vektoren transformiert sind, welche die erfindungsgemäßen Expressionseinheiten tragen. Erfindungsgemäß sind *E. coli*-Zellen als Wirtszellen bevorzugt.

Gemäß einer besonders bevorzugten Ausführungsform dient die Erfindung der rekombinanten Herstellung von PDGF-AB und diese enthaltende Arzneimittel. Erfindungsgemäß wurden erstmals die PDGF-Gene A und B gemeinsam in einer prokaryontischen Wirtszelle exprimiert mit dem Ziel, beide Genprodukte in denaturierter Form bereits in geeigneten Mengenverhältnissen für die nachfolgende *in vitro*-Rekonstitution zum biologisch aktiven Heterodimer zu gewinnen. Die Steuerung der Syntheseraten der PDGF-A- und PDGF-B-Monomere im Cytoplasma von *E. coli* entsprechend den stöchiometrischen Verhältnissen, die für eine nachfolgende *in vitro*-Dimerisierung zum AB-Heterodimer vorteilhaft sind, bietet die Grundlage zur Entwicklung neuartiger effektiver Präparationstechniken für dieses Dimer. Aufgrund ihres sehr ähnlichen Molekulargewichtes lassen sich die PDGF-A- und PDGF-B-Ketten mit Hilfe eines Gelfiltrationschromatographieschrittes **zusammen**, ohne das durch die erfindungsgemäße "genetische Titration" eingestellte Verhältnis der verschiedenen Ketten zueinander während der Präparationsprozedur zu variieren, weiter aufreinigen, renaturieren und zum biologisch aktiven PDGF-AB-Heterodimer rekonstituieren. Weitere aufwendige Arbeitsschritte wie die Probedimerisierung, mit der die optimalen Mengenverhältnisse der getrennt isolierten PDGF-Ketten A und B für die nachgeschaltete Renaturierung zum PDGF-AB eingestellt werden, können entfallen, da die Expressionshöhe beider PDGF-Ketten zueinander über die "genetische Titration", d.h. die individuelle Expressionshöhe bereits festgelegt ist. Andererseits ist es möglich, die Dimerisierung direkt, ohne weitere dazwischengeschaltete Reinigungsschritte mit dem gesamten solubilisierten Einschlußkörperprotein durchzuführen. Dieses Verfahren bietet den Vorteil, daß für alle weiteren notwendigen Reinigungs- und Analyseschritte PDGF-Dimere, die wegen ihrer besseren Wasserlöslichkeit leichter zu handhaben sind als die Monomere, eingesetzt werden können. Die PDGF-Dimere können nachfolgend zusammen mit üblichen Hilfs- und Trägerstoffen zu Arzneimitteln formuliert werden.

Im Zusammenhang mit der erfindungsgemäßen Herstellung PDGF-AB betrifft die Erfindung gemäß einer besonders bevorzugten Ausführungsform eine Expressionseinheit für die simultane und direkte Expression von für PDGF-A- und PDGF-B kodierenden DNA-Sequenzen in einer Bakterienzelle, welche gekennzeichnet ist durch die allgemeine Formel

$$p - TIS_1 - C_1 - TIS_2 - C_2 - t - r,$$

in der

"p" der $\lambda P_R$-Promotor ist,

"$C_1$" und "$C_2$" alternativ Sequenzen enthalten, die für PDGF-A oder PDGF-B kodieren und beide Gene gleichzeitig in der Expressionseinheit enthalten sind,

$TIS_1$ und $TIS_2$ jeweils die Sequenz TIS B (SEQ. ID. NO: 15) oder TIS E (SEQ. ID. NO: 14) sind und untereinander gleich oder verschieden sein können,

"t" der Terminator des Bakteriophagen fd ist und

"r" das cl857-Gen für den temperatursensitiven $\lambda$-Repressor ist.

In der erfindungsgemäßen Expressionseinheit zur Herstellung von PDGF-AB Heterodimeren können die Cistrons $C_1$ und $C_2$ alternativ die reife PDGF-B-Sequenz gemäß SEQ. ID. NO: 10, deren Analoga oder jegliches Fragment enthalten, welches für eine biologisch wirksame PDGF-B-Kette kodiert; insbesondere kommt in diesem Zusammenhang der zur maturen PDGF-B-Kette homologe Bereich des *v-sis*-Gens, das Produkt des Simian Sarcoma Virus, in Betracht, während das jeweils andere Cistron $C_1$ oder $C_2$ die lange oder kurze PDGF-A-Sequenz (PDGF-$A_L$ oder PDGF-$A_K$ gemäß SEQ. ID. NO: 12) sowie jegliche Fragmente enthalten, welche für eine biologisch aktive PDGF-A-Kette kodieren, d.h. eine der beiden Sequenzen $C_1$ oder $C_2$ enthält die PDGF-B-Sequenz und die jeweils andere die PDGF-A-Sequenz.

Besonders bevorzugt sind erfindungsgemäß Expressionseinheiten, in denen

a) "$TIS_1$" die TIS B Sequenz gemäß SEQ. ID. NO: 15, "$TIS_2$" die TIS E Sequenz gemäß SEQ. ID. NO: 14, "$C_1$" die PDGF-B-Sequenz gemäß SEQ. ID. NO: 10 und "$C_2$" die PDGF-A-Sequenz gemäß SEQ. ID. NO: 12 sind,

b) "$TIS_1$" und "$TIS_2$" die TIS B Sequenzen gemäß SEQ. ID. NO: 15, "$C_1$" die PDGF-B-Sequenz gemäß SEQ. ID. NO: 10 und "$C_2$" die PDGF-A-Sequenz gemäß SEQ. ID. NO: 12 sind oder

c) "$TIS_1$" und "$TIS_2$" die TIS B Sequenzen gemäß SEQ. ID. NO: 15 sind, "$C_1$" die PDGF-A-Sequenz gemäß SEQ. ID. NO: 12 und "$C_2$" die PDGF-B-Sequenz gemäß SEQ. ID. NO: 10 sind.

Die Erfindung schließt ferner Vektoren zur Verwendung bei der Herstellung von PDGF-AB ein, welche die zuvor erläuterten Expressioneinheiten enthalten. Ein besonders bevorzugter Vektor ist in Figur 1 und dessen Herstellung in Abschnitt 2 Punkt 1 dargestellt. Das Derivat pBS/PDGF-BA2 dieses Vektors (siehe unten) steuert die außerordentlich hohe Syntheserate von mehr als 150 mg PDGF-A- und PDGF-B-Ketten je Liter Kultur mittels einer Bakterienfeuchtmasse von 25 g/l. Dieser Vektor ist damit einer der leistungsfähigsten bekannten Expressionssysteme für *E. coli.* Dies gilt insbesondere in Anbetracht der Tatsache, daß es sich bei den PDGF-Ketten um kleine Proteine handelt; es werden demgemäß hohe Molmengen an rekombinantem Protein erzielt. Im Hochzelldichte-Fermentationsverfahren lassen sich mit Zellen, die das in Figur 1 dargestellte Plasmid tragen, noch weitaus höhere Erträge erreichen.

Die Erfindung betrifft ferner Bakterienzellen, welche mit einem Vektor wie oben beschrieben transformiert sind. Gemäß einer besonders bevorzugten Ausführungsform der Erfindung sind die Zellen mit einem Vektor gemäß Figur 1 transformiert. Bevorzugte Wirtszellen sind *E. coli*-Zellen. Die nachfolgend angegebenen erfindungsgemäß transformierten Zellen wurden am 4.6.1993 bei der Deutschen Sammlung für Mikroorganismen und Zellkulturen GmbH DSM hinterlegt:

1. TG2/pBS/PDGF-AB4 (DSM 8337)
2. TG2/pBS/PDGF-BA2 (DSM 8336)
3. TG2/pBS/PDGF-BA4 (DSM 8338)

Die Erfindung schließt weiterhin auch ein Verfahren zur Herstellung von heterodimerem rPDGF-AB durch direkte cytoplasmatische Coexpression der monomeren PDGF-Ketten A und B in prokaryontischen Wirtszellen ein, in dessen Verlauf *E. coli*-Zellen als Wirtszellen, welche die erfindungsgemäße Expressionseinheit operativ insertiert enthalten, in einem geeigneten Medium kultiviert werden und das so erzeugte rPDGF in der Form biologisch inaktiver monomerer Ketten A und B gemeinsam aus den baktierellen Einschlußkörpern isoliert und zum aktiven PDGF-AB Heterodimer renaturiert wird. Generell kommen im Rahmen der Erfindung als Medium alle bekannten Medien zum Kultivieren von Prokaryonten in Betracht, einschließlich synthetischer, proteinfreier oder proteinarmer Produktionsmedien. Erfindungsgemäß wurden TB- und LB-Medien bevorzugt. Die heteromeren, renaturierten Proteine können im nächsten Schritt mit üblichen Hilfs- und Trägerstoffen zu Arzneimitteln formuliert werden.

Die direkte gemeinsame multicistronische Expression der Untereinheiten eines heteromeren eukaryontischen Proteins in Form unabhängiger momomerer Komponenten im Cytoplasma einer prokaryontischen Wirtszelle ist bisher im Stand der Technik nicht bekannt; sie bietet gegenüber der bekannten monocistronischen Expression der Proteinuntereinheiten in verschiedenen Wirtszellen oder auf verschiedenen Plasmiden eine Reihe entscheidender verfahrenstechnischer Vorteile. Auf dem Hintergrund der erfindungsgemäßen Lehre, die in derartigen multicistronischen Expressionseinheiten die individuelle Regulierung der Translation von Fremdgenen erlaubt, wird erfindungsgemäß ein Verfahren geschaffen, welches von höchster Wirtschaftlichkeit ist. Wie anhand der erfindungsgemäß bevorzugten Ausführungsform der Herstellung von heterodimerem PDGF-AB gezeigt, kann die Expression der einzelnen Komponenten im Verhältnis von 1:1 erzielt werden und es muß zudem nur noch **ein** prokaryontischer Wirtsstamm kultiviert und validiert werden, da **beide** Ketten PDGF-A und PDGF-B mit Hilfe **einer** Fermenterkultur in großem Maßstab synthetisiert werden können.

Die Erfindung ist ganz generell dort anwendbar, wo die unterschiedlichen Protein-Untereinheiten heteromerer Proteine gemeinsam exprimiert und isoliert und/oder zum Multimer rekonstituiert werden sollen. Beispielsweise können auf diese Weise neben PDGF-AB Proteine wie VEGF, Scatter-Faktor (HGF-SF), Mitglieder der TGF-β Superfamilie, Bone Morphogenic Protein (BMP), Faktoren der Integrin/Cadherin Familie, Immunglobuline, Histokompatibilitätsantigene, Hämoglobin oder T-Zell Rezeptoren bzw. deren natürliche oder synthetische Varianten hergestellt werden.

Schließlich umfaßt die Erfindung ein Verfahren zur Herstellung von multimere Proteine enthaltenden pharmazeutischen oder kosmetischen Präparaten, wobei die nach dem erfindungsgemäßen Verfahren hergestellten multimeren Proteine mit den üblichen Hilfs- und Trägerstoffen formuliert werden. Gemäß einer besonders bevorzugten Ausführungsform der Erfindung enthalten die pharmazeutischen und/oder kosmetischen Präparate PDGF-AB, hergestellt nach dem erfindungsgemäßen Verfahren. Bei den pharmazeutischen oder kosmetischen Präparaten kann es sich um Salben, Sprays, Gele, Wundverbände, Pflaster und/oder Wundauflagen handeln, die nach im Stand der Technik bekannten Verfahren hergestellt werden.

Die Erfindung wird nachfolgend anhand von Beispielen erläutert:

Figur 1    Schematische Darstellung des Expressionsvektors pCYTEX P3.
TIS B=synthetische TIS (SEQ ID NO 15); fd=Transkriptions-Terminator des Bakteriophagen fd; cl857=Gen des Temperatur sensitiven Lambda-Repressors.

Figur 2    Schematische Darstellung der verschiedenen bicistronischen Konstrukte auf Basis von pCYTEX P3 PDGF-A=Gen für die reife humane PDGF-A-Kette; PDGF-B=Gen für die reife humane PDGF-B-Kette; $P_r=P_R$-Promotor des Bakteriophagen Lambda; TIS B=synthetische TIS (SEQ ID NO 15); TIS E=TIS des atpE-Gens aus pCYTEX P1 (SEQ ID NO: 14); fd=Transkriptions-Terminator des Bakteriophagen fd.

Figur 3    Einschlußkörperpräparationen nach Hoppe et al. (1989) aus Zellen des E. coli Stammes TG2 transformiert mit bicistronischen Konstrukten gemäß Figur 2.
Zur Analyse wurden Einschlußkörper aus 100 ml-Schüttelkulturen Plasmid-tragender Zellen aufgearbeitet und gleiche Mengen über SDS-Polyacrylamid-Gelelektrophorese nach Lämmli (1970) in Gegenwart von 5% Mercaptoethanol und unter Verwendung eines Gels mit 15% Acrylamid und 0,475% Bisacrylamid aufgetrennt. Nach dem Lauf wurde das Gel mit Coomassieblau gefärbt. Protein-Standard (1), Einschlußkörper aus Zellen mit pBS/PDGF-B (2), pBS/PDGF-A (3), pBS/PDGF-BA1 (4), pBS/PDGF-BA2 (5), pBS/PDGF-BA4 (6), pBS/PDGF-AB1 (7), pBS/PDGF-AB2 (8) und pBS/PDGF-AB4 (9).

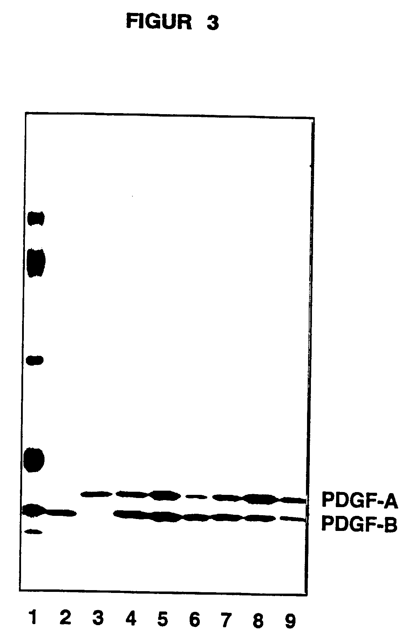

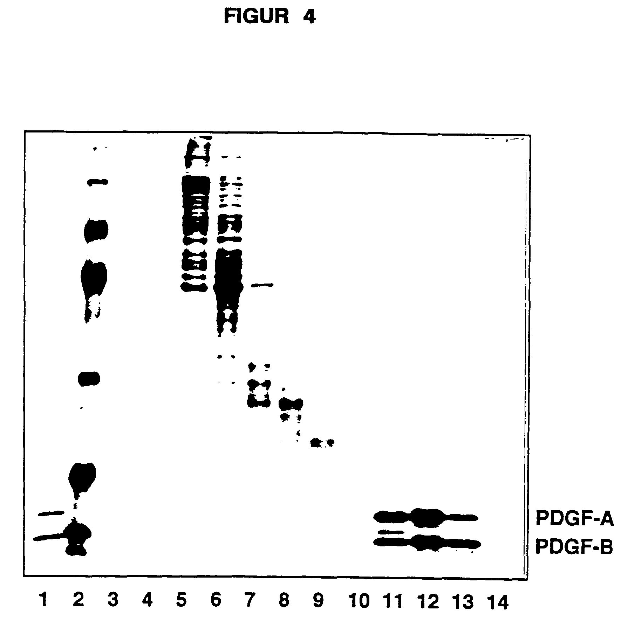

Figur 4    Gemeinsame Aufreinigung der PDGF-A- und PDGF-B-Monomere aus solubilisiertem Einschlußkörperprotein über Gelfiltration.
Gezeigt sind neben Protein-Standard (2) das auf die Säule aufgetragene rohe Einschlußkörperprotein aus Zellen mit dem Plasmid pBS/PDGF-BA2 (1) und die aus der Chromatographie resultierenden proteinhaltigen Fraktionen (3-14). Die PDGF-A- und PDGF-B-Ketten werden gemeinsam unter Beibehaltung des durch genetische Titration eingestellten stöchiometrischen Verhältnisses von 1:1 aufgereinigt (Spuren 11-13). Aufgrund der relativen Sauberkeit der PDGF-Ketten bereits auf der Stufe der Einschlußkörperpräparationen (1; vergleiche Figur 3 ist dieser eine Gelfiltrationsschritt ausreichend, um Proteinpräparationen einer Reinheit von ≥90% zu erzielen. Die Proben wurden über SDS-Polyacrylamid-Gelelektrophorese nach Lämmli (1970) in Gegenwart von 5% Mercaptoethanol und unter Verwendung eines Gels mit 15% Acrylamid und 0,475% Bisacrylamid aufgetrennt. Nach dem Lauf wurde das Gel mit Coomassieblau gefärbt.

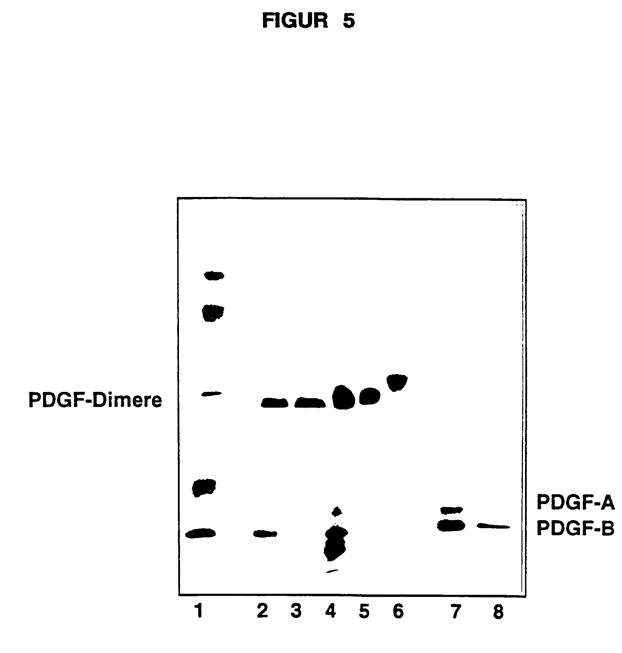

Figur 5    Dimerisierung der gemeinsam über Gelfiltration aufgereinigten PDGF-A- und PDGF-B-Monomere.
Protein-Standard (1); Dimerisierungsansatz mit gemeinsam über Gelfiltration aufgereinigten PDGF-A- und PDGF-B-Ketten (2; 7, nach Reduzierung der Probe mit 5% Mercaptoethanol); PDGF-AB-Heterodimer, von den Monomeren des Dimerisierungsansatzes über Ionenaustauschchromatographie abgetrennt (3, 8, nach Reduzierung der Probe mit 5% Mercaptoethanol); aus monocistronisch exprimierten und einzeln aufgereinigten Monomeren rekonstituiertes PDGF-AB (4), PDGF-BB (5) und PDGF-AA (6) als Kontrollen. Die Proben wurden über SDS-Polyacrylamid-Gelelektrophorese unter Verwendung eines Gels mit 15% Acrylamid und 0,475% Bisacrylamid aufgetrennt. Nach dem Lauf wurde das Gel mit Coomassieblau gefärbt.

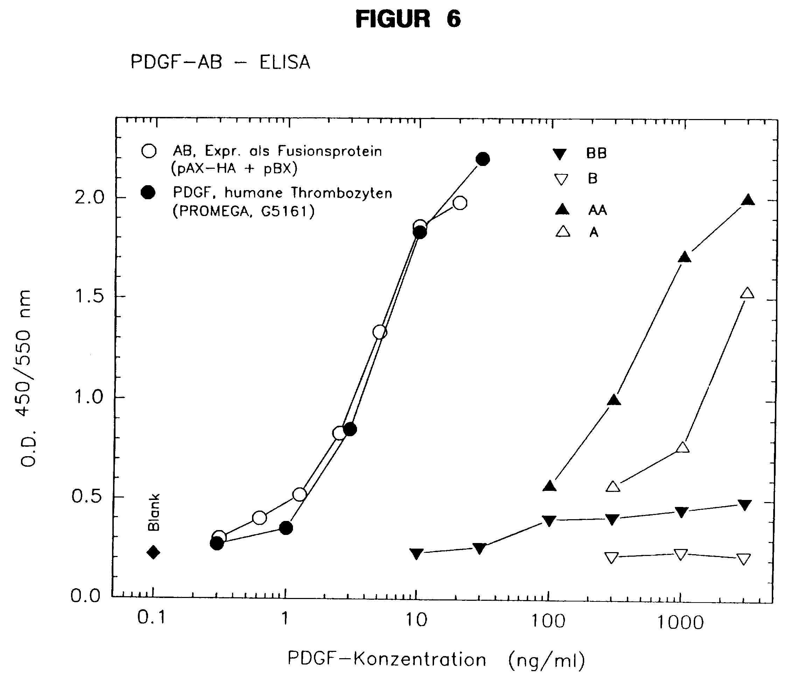

Figur 6    Sandwich-ELISA zum Nachweis von PDGF-AB mit Hilfe eines monoklonalen und eines polyklonalen Anti-PDGF-Antikörpers: Eichkurven von PDGF-Standards.

Polystyrolplatten wurden mit Schaf-Anti-Maus-IgG beschichtet und anschließend mit einem Maus-Hybridomaüberstand (von Klon 1B3, enthält monoklonale Antikörper gegen die B-Kette in PDGF-AB und -BB) inkubiert; nach Inkubation mit verschiedenen PDGF Standards wurde das gebundene PDGF mit Hilfe eines polyklonalen Kaninchen Anti-PDGF-AA, gefolgt von Peroxidase-markiertem Anti-Kaninchen-IgG nachgewiesen; Quelle der PDGF-Standards:

AB: aus humanen Thrombozyten, von PROMEGA Corp. No. G 5161;

oder analog WO 90/08163 aus *E. coli* (monocistronische Expression als Fusionsprotein für PDGF-A und PDGF-B). Die durch die Fusionsprotein-Expression gewonnene PDGF-B Kette weist im Gegensatz zu dem durch direkte Expression gewonnenen Material eine Verkürzung am N-Terminus um 12 Aminosäuren und eine C-terminale Verlängerung um 5 Aminosäuren auf. Bei der Fusionsgenexpression analog WO 90/08163 wurde anstelle des die PDGF-B-Kette enthaltenden Plasmids pE-pF14 (s.a. WO 90/04035) das Konstrukt pBX eingesetzt. Dieses Konstrukt führt zur Expression einer PDGF-B-Kette mit korrektem C-Terminus.

BB: rekombinant aus *E. coli* (pBS/PDGF-B)
B : rekombinant aus *E. coli* (pBS/PDGF-B)
AA: rekombinant aus *E. coli* (pBS/PDGF-A)
A : rekombinant aus *E. coli* (pBS/PDGF-A)

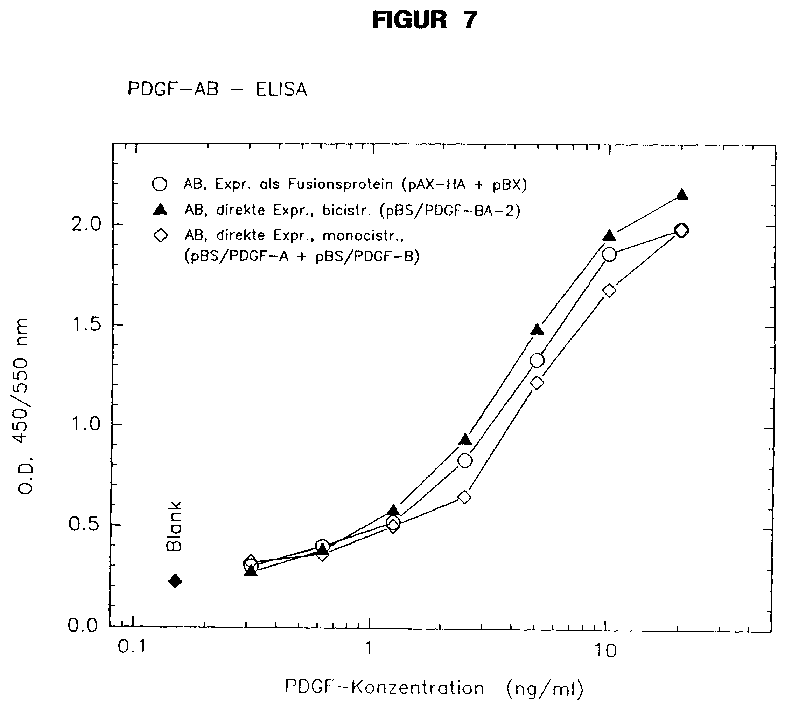

Figur 7    PDGF-AB-ELISA zur Analyse verschiedener PDGF-AB Präparationen. Untersucht wurden Präparationen aus unterschiedlichen mono- und bicistronischen PDGF-Expressionskonstrukten: (Eichkurven von Standards (siehe Figur 6).

**2. Beispiel: Steuerung der bicistronischen Expression der monomeren PDGF-Ketten A und B im Cytoplasma von *E. coli* und Rekonstitution der Monomere zum biologisch aktiven Heterodimer**

2.1 Herstellung des Expressionsvektors und der bicistronischen PDGF-Genkonstruktionen

2.1.1 Herstellung des Expressionsvektors pCYTEX P3

Zur Herstellung des Expressionsvektors, wie sie in Tabelle 1 beschrieben ist, und zur weiter unten (2.1.4) geschilderten Konstruktion der verschiedenen bicistronischen Plasmidkonstrukte und deren Vorstufen, wurden Standardmethoden der rekombinanten DNA-Technologie (Sambrook et al., 1989; Maniatis et al., 1982) eingesetzt.

Tabelle 1:

| Zusammensetzung des Expressionsvektors pCYTEX P3 | |
|---|---|
| SEQUENZ | HERKUNFT/KONSTRUKTION |
| Lambda P$_R$ Promotor | synthetisches Oligodesoxyribonukleotidpaar mit *Sma*I bzw. *Bgl*II kompatiblen Enden |
| TIS B | synthetisches Oligodesoxyribonukleotidpaar mit *Bgl*II bzw. *Nde*I kompatiblen Enden |
| fd Terminator | Terminator des Bakteriophagen fd nach Subklonierungen als Smal/Xbal Fragment eingebaut |
| *cl*857 Repressorgen | nach Einfügung einer *Kpn*I-Schnittstelle stromauf-wärts von dessen Promotorsequenz mittels <u>in vitro</u> Mutagenese als *Kpn*I/*Not*I Fragment aus pCYTEXP1 eingebaut |
| Ampicillin-Resistenzgen *Nde*I/*Pvu*II-Fragment aus pUC18 und Replikationsursprung | |

Nach dem Einbau eines *Not*I/*Sma*I-Adaptors zwischen die *Nde*I und *Eco*RI Schnittstellen, wodurch diese zerstört worden sind, wurde die subklonierte Expressionskassette zwischen die *Not*I und *Pvu*II Schnittstellen in pUC18 insertiert. Die schematische Darstellung des Vektors ist in Figur 1 und die Sequenz von dessen Expressionskassette inklusive flankierender Bereiche ist in SEQ ID NO: 16 dargestellt. In diesem Vektor erfolgt die Transkription eines zwischen den Schnittstellen *Nde*I bis *Xba*I eingefügten Gens vom $P_R$-Promotor des Bakteriophagen Lambda aus und wird am Transkriptions-Terminator des fd Phagen beendet. Das zur Kontrolle der Transkription notwendige *cI*857-Gen, das für den Temperatur-sensitiven Repressor des Lambda-Promotors kodiert, ist ebenfalls auf dem Vektor enthalten, um die Verwendung dieses Expressionsvektors unabhängig vom Vorhandensein eines chromosomal kodierten Repressors in der Zelle zu machen. Die Transkription der unter der Kontrolle des Lambda $P_R$-Promotors befindlichen Gene ist in Zellen, die bei 30° C kultiviert werden, reprimiert und wird nach Inaktivierung des Repressors durch Erhöhung der Inkubationstemperatur auf 40-42° C effektiv induziert. Zur Sicherstellung der effektiven Translations-Initiation läßt sich ein rekombinantes Gen über die *Nde*I Schnittstelle mit seinem Startcodon an die synthetische TIS B des Vektors anschließen.

2.1.2 Mutagenese am humanen PDGF-B-Gen

Das Plasmid pMVW-2 enthält die cDNA des humanen PDGF-B Gens, welches im 5'-translatierten Bereich der Vorläufersequenz unvollständig ist (Weich et al., 1986; SEQ ID NO: 1). Für die direkte Expression in *E. coli* wurde durch *in vitro* Mutagenesen an das 5'-Ende des reifen PDGF-B-Gens eine *Nde*I-Schnittstelle (enthält die Information für die Startaminosäure Methionin) und Stop-Codons an das 3'-Ende des maturen PDGF-B Gens eingeführt. Hierfür wurde zunächst das 914 bp *Bam*HI/*Nco*I-Fragment aus pMVW-2 über einen synthetischen Adapter [Oligomere NCCLSA1 und NCCLSA2 (SEQ ID NO: 5 + 6)] in den *Bam*HI/*Sal*I-gespaltenen Bakteriophagen M13mp19 (Pharmacia) insertiert. Dieses Konstrukt lieferte die notwendige Einzelstrang-DNA für die nachgeschalteten *in vitro* Mutageneseschritte, die mit Hilfe des Oligomer-directed *in vitro* mutagenesis system (Version 2) der Fa. Amersham, basierend auf der Methode von Eckstein et al. [Taylor J. W., Ott J. and Eckstein F. (1985) Nucl. Acids Res. **13**, 8764-8785; Nakamaye K. and Eckstein F. (1986) Nucl. Acids Res. **14**, 9679-9698; Sayers J. R., Schmidt W. and Eckstein F. (1988) Nucl. Acids Res. **16**, 791-802] durchgeführt wurden. Als Mutageneseprimer wurden die synthetischen Primer PDGBNDE und PDGBTGA (SEQ ID NO: 7 und 8) eingesetzt. Als Mutageneseprodukt wurde die aminoterminal um ein zusätzliches Methionin verlängerte Sequenz der maturen PDGF-B Kette (SEQ ID NO: 10) in M13/PDGF-B erhalten.

2.1.3 Mutagenese am humanen PDGF-A-Gen

Das Plasmid pODA (Eichner et al., 1989) enthält die vollständige Sequenz des humanen PDGF-A Precursors (SEQ ID NO: 3). Für die direkte Expression in *E. coli* wurde durch *in vitro* Mutagenese an das 5'-Ende des reifen PDGF-A-Gens eine *Nde*I-Schnittstelle (enthält die Information für die Startaminosäure Methionin) eingeführt. Hierfür wurde pODA zunächst mit EcoRI linearisiert, die resultierenden Enden mit Hilfe des Klenow-Fragmentes der DNA-Polymerase I von *E. coli* zu glatten Enden aufgefüllt und das nach Verdau mit *Bam*HI entstehende Fragment in den Expressionsvektor pCYTEX P1 insertiert. Dieser war zunächst mit *Sph*I geschnitten, dann mit Mung bean Nuklease behandelt und darauf mit *Bam*HI geschnitten worden. Mit Hilfe des Helferphagen M13K07 wurde von diesem Konstrukt, pCYT-A-1, die Einzelstrang-DNA für den nachgeschalteten *in vitro* Mutageneseschritt präpariert (Sambrook et al., 1989), der mit Hilfe des Oligomer-directed *in vitro* mutagenesis system (Version 2) der Fa. Amersham, basierend auf der Methode von Eckstein et al. [Taylor J. W., Ott J. and Eckstein F. (1985) Nucl. Acids Res. **13**, 8764-8785; Nakamaye K. and Eckstein F. (1986) Nucl. Acids Res. **14**, 9679-9698; Sayers J. R., Schmidt W. and Eckstein F. (1988) Nucl. Acids Res. **16**, 791-802] durchgeführt wurde. Als Mutageneseprimer wurde der synthetische Primer PDGANDE (SEQ ID NO: 9) eingesetzt. Als Mutageneseprodukt wurde die aminoterminal um ein zusätzliches Methionin verlängerte Sequenz der maturen PDGF-A Kette (SEQ ID NO: 12) in pCYT-A-2 erhalten.

2.1.4 Herstellung der bicistronischen PDGF-Genkonstrukte

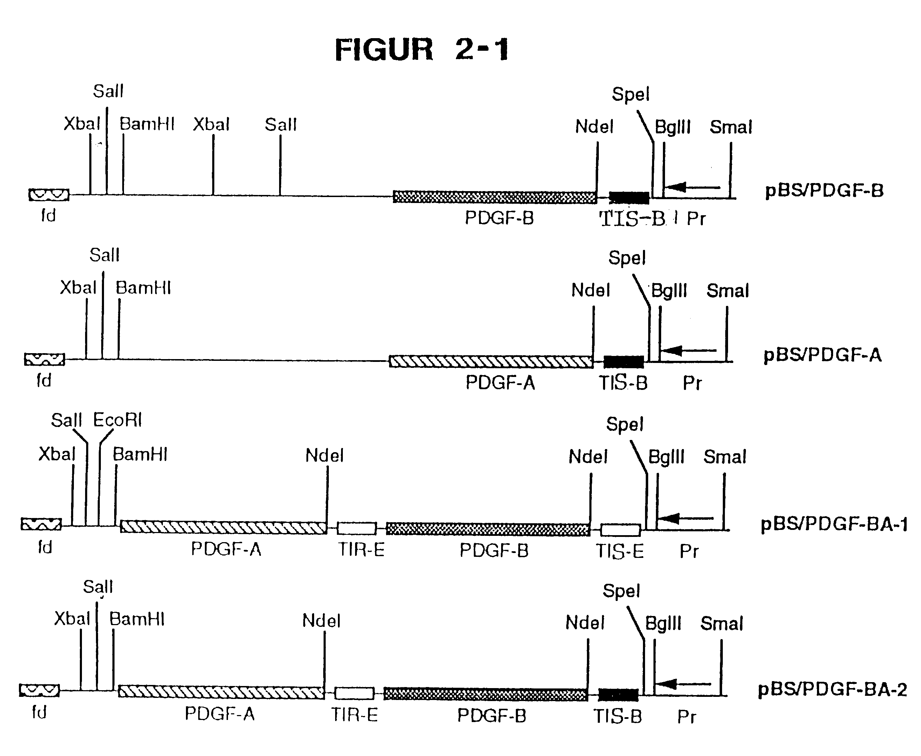

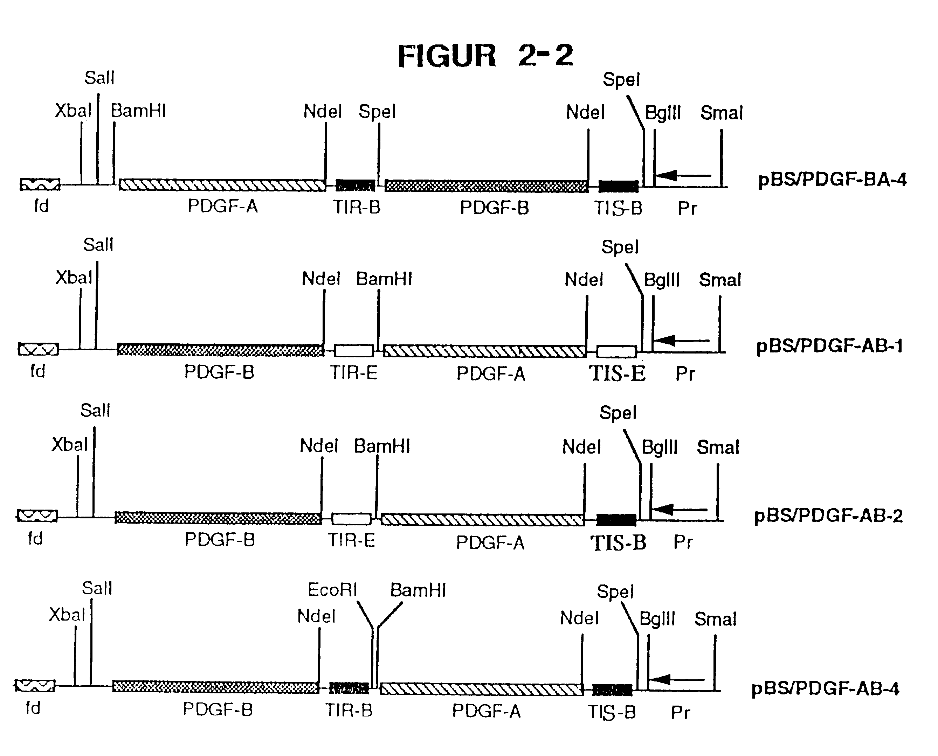

Basierend auf diesem Vektorsystem wurden die verschiedenen in Figur 2 dargestellten bicistronischen PDGF-Genkonstrukte hergestellt. Die Konstruktion dieser Plasmide wurde inklusive der verschiedenen Subklonierungen wie in Tabelle 2 beschrieben durchgeführt. Alle Klonierungen wurden mittels DNA-Sequenzierung überprüft.

Tabelle 2:

| Plasmidkonstruktionen | |
|---|---|
| PLASMID | KONSTRUKTION |
| pCYT-A3 | Entfernung des nicht gewünschten PDGF-A-Vorläufergenanteils durch *Nde*I Verdau von pCYT-A2 und Religation des Vektorfragmentes, wobei PDGF-A an die TIS E-Sequenz des Vektoranteils angeschlossen wurde |
| pCYT-B | Klonierung des *Nde*I/*Sal*I-Fragmentes mit der Sequenz für die reife PDGF-B-Kette aus M13/PDGF-B in pCYTEX P1 geschnitten mit *Nde*I und *Sal*I, wobei PDGF-B an die TIS E-Sequenz des Vektoranteils angeschlossen wurde |
| pCYT-BA | Klonierung des durch vollständige Spaltung mit *Bam*HI und partielle Spaltung mit *Nde*I entstandenen Fragmentes aus pBS/PDGF-BA2 mit PDGF-B ohne TIS gefolgt von PDGF-A mit TIS E in pCYTEX P1, wobei PDGF-B an die TIS E-Sequenz des Vektoranteils angeschlossen wurde |
| pCYT-B2 | Klonierung des *Xho*I/*Sal*I-Fragmentes mit PDGF-B aus pCYT-B in pCYTEX P1 geschnitten mit *Eco*RI und *Sal*I, wobei sowohl die *Xho*I - als auch die *Eco*RI-Enden der DNA-Fragmente zuvor mittels des Klenow-Enzyms zu glatten Enden aufgefüllt wurden |
| pCYT-B3 | Klonierung des *Spe*I/*Sal*I-Fragmentes mit PDGF-B aus pBS/PDGF-B in pCYTEX P1 geschnitten mit *Eco*RI und *Sal*I, wobei sowohl die *Spe*I - als auch die *Eco*RI-Enden der DNA-Fragmente zuvor mittels des Klenow-Enzyms zu glatten Enden aufgefüllt wurden |
| pCYT-AB1 | Klonierung des *Xho*I/*Bam*HI-Fragmentes mit PDGF-A inklusive TIS E aus pCYT-A3 in pCYT-B2 geschnitten mit *Xho*I und *Bam*HI |
| pCYT-AB2 | Klonierung des *Spe*I/*Bam*HI-Fragmentes mit PDGF-A inklusive TIS B aus pBS/PDGF-A in pCYT-B2 geschnitten mit *Xho*I und Bam*HI,* wobei sowohl die *Spe*I - als auch die *Xho*I-Enden der DNA-Fragmente zuvor mittels des Klenow-Enzyms zu glatten Enden aufgefüllt wurden. |
| pCYT-AB4 | Klonierung des *Spe*I/*Bam*HI-Fragmentes mit PDGF-A inklusive TIS B aus pBS/PDGF-A in pCYT-B3 geschnitten mit *Xho*I und *Bam*HI, wobei sowohl die *Spe*I - als auch die *Xho*I-Enden der DNA-Fragmente zuvor mittels des Klenow-Enzyms zu glatten Enden aufgefüllt wurden. |
| pBS/PDGF-B | Klonierung des PDGF-B-Gens aus pCYT-B als *Nde*I/*Xba*I-Fragment in vollständig mit *Nde*I und partiell mit *Xba*I gespaltenen pCYTEX P3, Anschluß an TIS B |
| pBS/PDGF-A | Klonierung des PDGF-A-Gens aus pCYT-A3 als *Nde*I/*Bam*HI-Fragment in pCYTEX P3 geschnitten mit *Nde*I und *Bam*HI, Anschluß an TIS B |
| pBS/PDGF-BA1 | Klonierung des *Xho*I/*Bam*HI-Fragmentes aus pCYT-BA mit PDGF-B inklusive TIS E gefolgt von PDGF-A ebenfalls mit TIS E in pCYTEX P3 geschnitten mit *Spe*I und *Bam*HI, wobei sowohl die *Spe*I - als auch die *Xho*I-Enden der DNA-Fragmente zuvor mittels des Klenow-Enzyms zu glatten Enden aufgefüllt wurden. |
| pBS/PDGF-BA2 | Klonierung des PDGF-A-Gens inklusive TIS E als *Xho*I/*Bam*HI-Fragment aus pCYT-A3 in vollständig mit *Bam*HI und partiell mit *Sal*I geschnittenen pBS/PDGF-B |
| pBS/PDGF-BA4 | Klonierung des PDGF-A-Gens inklusive TIS B aus pBS/PDGF-A als *Spe*I/*Bam*HI-Fragment in vollständig mit *Bam*HI und partiell mit *Sal*I geschnittenen pBS/PDGF-B, wobei sowohl die *Spe*I - als auch die *Sal*I-Enden der DNA-Fragmente zuvor mittels des Klenow-Enzyms zu glatten Enden aufgefüllt. |

Tabelle 2: (fortgesetzt)

| Plasmidkonstruktionen | |
|---|---|
| PLASMID | KONSTRUKTION |
| pBS/PDGF-AB1 | Klonierung des *Xho*I/*Xba*I-Fragmentes mit dem PDGF-A-Gen inklusive TIS E gefolgt von PDGF-B ebenfalls mit TIS E aus pCYT-AB1 in pCYTEX P3 geschnitten mit *Spe*I und *Bam*HI, wobei sowohl die *Spe*I - als auch die *Xho*I-Enden der DNA-Fragmente zuvor mittels des Klenow-Enzyms zu glatten Enden aufgefüllt wurden. |
| pBS/PDGF-AB2 | Klonierung des *Spe*I/*Xba*I-Fragmentes mit dem PDGF-A-Gen inklusive TIS B gefolgt von PDGF-B mit TIS E aus pCYT-AB2 in pCYTEX P3 geschnitten mit *Spe*I und *Bam*HI |
| pBS/PDGF-AB4 | Klonierung des *Spe*I/*Xba*I-Fragmentes mit dem PDGF-A-Gen inklusive TIS B gefolgt von PDGF-B ebenfalls mit TIS B aus pCYT-AB1 in pCYTEX P3 geschnitten mit *Spe*I und *Bam*HI |

## 2.2 Anzucht und Aufarbeitung der Zellen

### 2.2.1 Anzucht Plasmid tragender Zellen

*E. coli* Zellen transformiert mit einem der bicistronischen Plasmide wurden auf LB - oder TB Medium mit 0,05-0,1 mg/ml Ampicillin zunächst bei 30° C angezogen, um den Promotor des Bakteriophagen Lambda durch die Wirkung des bei dieser Temperatur aktiven Repressors im inaktiven Zustand zu halten. Nach Erreichen der gewünschten Zelldichte in den Kulturen in der Phase logarithmischen Wachstums wurde die Kultivierungstemperatur auf 42° C erhöht und damit die Expression der PDGF-Gene induziert. Im Falle von Schüttelkulturen wurde bei einer optischen Dichte von ca. 0,8 und für Fermenterkulturen bei ca. 10 induziert. Die Zellen wurden spätestens beim Erreichen der stationären Wachstumsphase durch Zentrifugation für 20 min bei 5000 x g geerntet.

### 2.2.2 Präparation von Einschlußkörpern

Die Präparation der Einschlußkörper wurde analog Hoppe et al. (1989) durchgeführt. Dabei wurden die nach 2.2.1 geernteten Zellen in 20 ml pro Liter Kultur einer 20 mM Tris-HCL/0,5 mM EDTA, pH 7,8 aufgenommen. Abweichend von dieser Methode erfolgte der Zellaufschluß mittels Ultraschallbehandlung. Die Einschlußkörper wurden durch 10-minütige Zentrifugation bei 6000 x g geerntet und einmal mit 20 mM Tris-HCl, 0,5 mM EDTA, 2% Triton X-100 gewaschen.

Durch die Verwendung des pCYTEX P3-Expressionssystems zur direkten cytoplasmatischen Expression der PDGF-Gene ließen sich Syntheseraten der reifen PDGF-A- und PDGF-B-Ketten erzielen, die zur Bildung von Einschlußkörpern im Cytoplasma führen. Die hier dargestellte hohe Reinheit der PDGF-A- und PDGF-B-Ketten (etwa 90%) bereits auf der Stufe des Einschlußkörperpräparationen offenbart hier einen gravierenden Vorteil der direkten cytoplasmatischen Expression der entsprechenden Gene mittels des Expressionsvektors pCYTEX P3 gegenüber der von Hoppe et al. (1989, s. a. WO 90/04035; 1990, s. a. WO 90/08163) eingesetzten Fusionsgenmethode. Beim letztgenannten Verfahren entstehen durch die Abspaltung des nicht gewünschten bakteriellen Fusionsgenanteils Proteinbruchstücke im Molekulargewichtsbereich der PDGF-Monomere. Fragmente dieser Art entstehen bei der direkten cytoplasmatischen Expression nicht.

Densitometrische Untersuchungen von Coomassieblau gefärbten Gelen mit den verschiedenen Einschlußkörper-Präparationen ergaben für die bicistronischen Konstrukte die in Tabelle 3 aufgeführten Verhältnisse von PDGF-A- zu PDGF-B-Monomeren. Für Zellen mit dem Plasmid pBS/PDGF-BA2 (Figur 2, DSM-Hinterlegungsnummer 8336) zeigt es sich, daß beide reifen Ketten, PDGF-B und PDGF-A, mit gleicher Rate effektiv synthetisiert und in Einschlußkörpern deponiert werden. Dieses Plasmid bietet damit verfahrenstechnische Vorteile. **Beide** Ketten, PDGF-A und PDGF-B, können mit Hilfe **einer** Fermenterkultur im großen Maßstab hergestellt werden. Damit bräuchte nur **ein** genetisch veränderter Organismus charakterisiert und nur **ein** Produktionsverfahren für **beide** Ketten validiert zu werden. Von den anderen bicistronischen Plasmiden werden jeweils zum Teil deutlich voneinander abweichende Expressionsraten des PDGF-A- und des PDGF-B-Gens gesteuert. Insbesondere kann mittels der Konstrukte pBS/PDGF-BA4 (DSM-Hinterlegungsnummer DSM 8338) bzw. pBS/PDGF-AB4 (DSM Hinterlegungsnummer DSM 8337, vgl. Figur 2), welche das PDGF-A- und das PDGF-B-Gen in den zwei möglichen Anordnungen jeweils mit der TIS B-Sequenz fusioniert tragen, einesfalls deutlich mehr PDGF-B- als PDGF-A-Kette und andernfalls mehr PDGF-A- als PDGF-B-Kette synthetisiert werden.

Tabelle 3:

| Bicistronische Expression von PDGF-A und PDGF-B | |
| --- | --- |
| | Verhältnis PDGF-A : PDGF-B |
| pBS/PDGF-BA1 | 0,77 |
| pBS/PDGF-BA2 | 0,97 |
| pBS/PDGF-BA4 | 0,45 |
| pBS/PDGF-AB1 | 0,9 |
| pBS/PDGF-AB2 | 3,7 |
| pBS/PDGF-AB4 | 3,6 |

### 2.3 Reinigung der PDGF Monomere und Rekonstitution zu PDGF-AB

Nachdem das Einschlußkörperprotein nach Hoppe et al. (1989) solubilisiert und sulfoniert worden war, wurde es über Gelfiltration aufgetrennt. Erfindungsgemäß bevorzugt wurde für die Aufreinigung der PDGF-A- und PDGF-B-Ketten eine Superdex 75 HR High Load 26/60-Säule von Pharmacia. Nach Äquilibrierung mit dem Laufmittel 4 M Guanidinium-HCl, 50 mM Tris-Cl, pH 7,4 wurden 5-10 ml Probe bei einer Flußrate von 2 ml/min aufgetragen. Wie in Figur 4 gezeigt wurden die PDGF-A- und die PDGF-B-Kette aufgrund ihres sehr ähnlichen Molekulargewichts mit annähernd ≥90% Reinheit in den gleichen Säulenfraktionen erhalten. Die Fraktionen wurden daraufhin vereinigt und über Nacht gegen 5 1 Wasser dialysiert. Nach dem Einstellen des Probenvolumens auf 15 ml durch Eintrocknen unter Vakuum erfolgte durch Zugabe von Ameisensäure und Harnstoff in Endkonzentrationen von 5% bzw. 5 M die Solubilisierung des ausgefallenen Proteinmaterials. Nach erneuter Dialyse gegen 5 1 2 M Harnstoff wurde die PDGF-haltige Probe auf ca. 0,5 mg/ml eingestellt. Anschließend erfolgte die Dimerisierung wie bei Hoppe et al. (1989) beschrieben. Wie in Figur 5 gezeigt ist, wurde dabei nach elektrophoretischer Auftrennung des Dimerisierungsansatzes neben Monomerbanden das Auftreten von einer prominenten Bande im Molekulargewichtsbereich von ca. 28000 beobachtet. Diese zeigt das gleiche Laufverhalten im Gel wie PDGF-AB und ein deutlich anderes als PDGF-AA- und PDGF-BB-Dimere, die jeweils zur Kontrolle aus einzeln aufgereinigten Ketten rekonstituiert und durch verschiedene Analysen verifiziert wurden. Ein Vergleich der Intensitäten der Dimerbande und der Monomerbanden des elektrophoretisch aufgetrennten Dimerisierungsansatzes deutet eine Dimerisierungsrate von mehr als 50% an.

Das Dimerisierungsprodukt wurde anschließend von den Monomeren über Ionenaustauschchromatographie wie bei Hoppe et al. (1990) beschrieben mittels Fraktogel TSK SP 650(S) der Firma Merck abgetrennt. Das gereinigte Dimer (Figur 5) wurde für weitere Analysen eingesetzt.

An dieser Stelle wird deutlich, daß das bicistronische Plasmid pBS/PDGF-BA2 auch die Grundlage zur Entwicklung neuartiger effektiver Präparationstechniken für das PDGF-AB-Heterodimer bietet. Dieses Plasmid steuert die Synthese der PDGF-A und PDGF-B Monomere in einem stöchiometrischen Verhältnis von 1:1 (Figur 3, Tabelle 3), welches besonders günstig ist für deren *in vitro* Dimerisierung zu PDGF-AB. Aufgrund ihres sehr ähnlichen Molekulargewichtes lassen sich die PDGF-A- und PDGF-B-Ketten mit Hilfe eines Gelfiltrations-Chromatographieschrittes **zusammen**, ohne das durch genetische Titration eingestellte Verhältnis der verschiedenen Ketten zueinander während der Präparationsprozedur zu variieren, weiter aufreinigen, renaturieren und zum biologisch aktiven PDGF-AB-Heterodimer rekonstituieren. Aufgrund der hohen Reinheit der PDGF-Ketten bereits auf der Stufe der Einschlußkörperpräparationen bietet sich andererseits die Möglichkeit an, die Dimerisierung direkt ohne weitere Reinigungsschritte dazwischenzuschalten mit dem gesamten solubilisierten Einschlußkörperprotein durchzuführen. Der wesesentliche Vorteil dieses Verfahren wäre, daß für alle weiteren notwendigen Reinigungs- und Analyseschritte PDGF-Dimere eingesetzt werden könnten, die wegen ihrer besseren Wasserlöslichkeit leichter zu handhaben sind als die PDGF-Monomere.

### 2.4 Nachweis von PDGF-AB Heterodimer in den Renaturierungsproben mit Hilfe eines spezifischen PDGF-AB ELISA's

Es wurde ein 'two-antibody sandwich assay' aufgebaut, der eine spezifische Quantifizierung von PDGF-AB neben PDGF-AA und -BB erlaubt. Dabei wurde ein monoklonaler und ein polyklonaler anti-PDGF-Antikörper eingesetzt:

Polystyrolplatten mit 96 Kavitäten (Fa. Dynatech, U-Platte, No. M124B) werden in folgender Reihenfolge beschichtet (zwischen jedem Schritt jeweils 4 x Waschen mit PBS mit 0,05% Tween 20):

1) Maus Monoklonaler Antikörper 1B3 [erhalten aus Überständen von Hybridomazellen, die durch Fusion von SP2/0-Myelomzellen mit Milzzellen von Mäusen entstanden waren. Die Mäuse waren mit rekomb. PDGF-AB (aus *E. coli* gemäß Hoppe et al., 1990) immunisiert. Der monoklonale Antikörper bindet spezifisch an der B-Kette von PDGF-Dimeren], 3 µg/ml IgG2a in Carbonat/Bicarbonat-Puffer, pH 9,6, 50 µl bei 4°C über Nacht.

2) 1% BSA (Fa. E. Merck, Nr. 12018) in PBS, pH 7,5, 100 µl für 1 h bei R.T.

3) PDGF-haltige Lösungen, verdünnt in PBS mit 0,1 % BSA und 0,05 % Tween 20 (PBS+), 50 µl für 1 h bei R.T.

4) Polyklonales Kaninchen-Anti-PDGF-AA-IgG (Fa. Genzyme, No. ZP-214, bindet an der A-Kette von dimerem PDGF), 2 µg/ml in PBS+, 50 µl für 1 h bei R.T.

5) POD-markiertes Ziege-Anti-Kaninchen IgG (Fa. Pierce, No. 31460), 0,1 µg/ml in PBS+, 50 µl für 1 h bei R.T., Detektion mit dem Substrat Tetramethylbenzidin gemäß E.S. BOS et al. (J.Immunoassay 2 (1981), 187-204).

2.5 Biologische Aktivität von renaturiertem und gereinigtem rPDGF-AB

Die Messung der Stimulierung der DNA-Syntheserate von dichtearretierten Fibroblasten erlaubt eine Bestimmung der mitogenen Aktivität des PDGF. Eine Unterscheidung zwischen den Isoformen PDGF-AA, AB und BB ist nicht möglich, da alle PDGF-Isoformen in diesem Testsystem aktiv sind.

Der Assay wurde gemäß Shipley et al. (1984) mit AKR-2-B-Mausfibroblasten in 24-Well-Platten durchgeführt.

Reines PDGF zeigt in dem Test eine halbmaximale Stimulierung bei einer Konzentration von etwa 3-5 ng/ml. Die Ergebnisse aus dem Mitogentest sind in Tabelle 4 den aus dem PDGF-AB-ELISA abgeleiteten $EC_{50}$-Werten (Konzentration, bei der ein halbmaximaler Effekt auftritt) gegenübergestellt. Die Meßdaten für rekombinantes PDGF-AB aus mono- oder bicistronischer Expression sind annähernd identisch.

Tabelle 4

| Plasmid | mono-cis. | bi-cis. | $EC_{50}$ gemäß PDGF-AB ELISA [µg/ml] | Unit gemäß Mitogentest [µg/ml] |
|---|---|---|---|---|
| pAX-HA +pBX (Expression als Fusionsprotein) | + | - | 4 | 5.4 |
| pBS/PDGF-B+ pBS/PDGF-A (direkte Expression) | + | - | 5 | 3.0 |
| pBS/PDGF-BA2 (direkte Expression) | - | + | 3.3 | 3.9 |

2.6 Aminoterminale Sequenzierung von PDGF-Polypeptiden

Durch diese Untersuchungen sollte im renaturierten, durch cytoplasmatische Coexpression erhaltenen PDGF-Dimer die PDGF-Ketten A und B eindeutig nebeneinander nachgewiesen werden. Das für diese Analysen eingesetzte Material wurde mit Hilfe des bicistronischen Konstruktes pBS/PDGF-BA2 (Tabelle 2) erhalten.

Die automatische Sequenzanalyse wurde am Modell 477A (Applied Biosystems) mit dem Zyklus NORMAL1 durchgeführt. Die Analyse der Phenylthiohydantoin-Aminosäuren-Derivate erfolgte auf dem online gekoppelten 120A PTH-Analyser. Die Disulfidbrücken der Probe wurden mit Dithiothreitol reduziert und mit 4-Vinylpyridin alkyliert.

Nach den Ergebnissen der Proteinbestimmung wurden 10 µg Probe analysiert. Die Ergebnisse der Proteinsequenzanalyse von coexprimierten PDGF-Monomeren (Tabelle 5) zeigen, daß die N-terminalen Aminosäuren der PDGF-A- und PDGF-B-Kette in annähernd gleicher Ausbeute nachgewiesen werden können. Dies ist ein zusätzlicher Beleg dafür, daß die Expression mit dem patentgemäß bevorzugten Konstrukt pBS/PDGF-BA2 zu einem stöchiometrischen 1:1 Verhältnis beider PDGF-Ketten führt mit der Folge, daß durch die Anwendung der in Abschnitt 2.3 beschriebenen Präparationsmethode hauptsächlich PDGF-AB-Heterodimere erhalten werden. Die relative Ausbeute der Aminosäure Serin in Position 1 wurde mit ≥96% bestimmt. Nebensequenzen (z. B. mit N-terminalem Methionin versehene PDGF-Ketten) machen daher weniger als 4% des Gesamtproteins aus.

Tabelle 5

| Aminosäuresequenzanalyse der PDGF-A- und PDGF-B-Ketten | | | | |
|---|---|---|---|---|
| | PDGF-A | | PDGF-B | |
| Zyklus | Code | Ausbeute (pmol) | Code | Ausbeute (pmol) |
| 1 | Ser | 33,11* | Ser | |
| 2 | Ile | 128,58 | Leu | 109,07 |
| 3 | Glu | 61,47 | Gly | 71,95 |
| 4 | Glu | 44,82 | Ser | 24,93 |
| 5 | Ala | 72,32 | Leu | 73,41 |
| 6 | Val | 80,22 | Thr | 33,90 |
| 7 | Pro | 54,00 | Ile | 44,88 |
| 8 | Ala | 53,49* | Ala | |
| 9 | Val | 65,06 | Glu | 30,51 |

\* Ausbeute aus beiden Ketten

## LITERATUR

Bassford Jr., P. J., Silhavy, T. J. and Beckwith, J. R. (1979) J. Bacteriol. **139**, 19-31.

Belev, T. N., Singh, M. and McCarthy, J. E. G. (1991) Plasmid **26**, 147-150.

Betsholtz, C., Johnsson, A., Heldin, C.-H., Westermark, B., Lind, P., Urdea, M. S., Eddy, R., Shows, T. B., Philpott, K., Mellor, A. L., Knott, T. J. and Scott, J. (1986) Nature **320**, 695-699.

Better, M., Chang, C. P., Robinson, R. R. and Horwitz, A. H. (1988) Science **240**, 1041-1043.

Block, L. H., Emmons, L. R., Vogt, E., Sachinidis, A., Vetter, W. and Hoppe, J. (1989) Proc. Natl. Acad. Sci. USA **86**, 2388-2392.

Boss, M. A., Kenten, J. H., Wood, C. R. und Emtage, J. S. (1984) Nucleic Acids Res. **12**, 3791-3806.

Brewer, S. J. and Sassenfeld, H. M. (1985) Trends Biotechnol. **3**, 119-122.

Brosius, J. (1984) Gene **27,** 161-172.

Buell, G. und Panayotatos, N. (1986) in Maximizing Gene Expression (Reznikoff, W. and Gold, L., eds.) pp 345-363, Butterworths, Boston.

Buell, G., Schulz, M.-F., Selzer, G., Chollet, A., Movva, N. R., Semon, D., Escanez, S. and Kawashima, E. (1985) Nucleic Acids Res. **13,** 1923-1938.

Cabilly, S., Riggs, A. D., Pande, H., Shively, J. E., Holmes, W. E., Rey, M., Perry, L. J., Wetzel, R. und Heynecker, H. L. (1984) Proc. Natl. Acad. Sci. USA **81,** 3273- 3277.

Cabradilla, C. D., Groopman, J. E., Lanigan, J., Renz, M., Lasky, L. A. and Capon, D. J. (1986) Biotechnology **4,** 128-133.

Carter, P., Kelley, R. F., Rodrigues, M. L., Snedecor, B., Covarrubias, M., Velligan, M. D., Wong, W. L. T., Rowland, A. M., Kotts, C. E., Carver, M. E., Yang, M., Bourell, J. H., Shepard, H. M. and Henner, D. (1992) Biotechnology **10**, 163-167.

Chan, S. J., Weiss, J., Konrad, M., White, T., Bahl, C., Yu, S.D., Marks, D. and Steiner, D. F. (1981) Proc. Natl. Acad. Sci. USA **78,** 5401-5405.

Chang, C. N., Rey, M., Bochner, B., Heynecker, H. and Gray, G. (1987) Gene **55,** 189-196.

Crowl, R., Seamans, C., Lomedico, P. and McAndrew, S. (1985) Gene **38**, 31-38.

Dalboge, H., Bech Jensen, E., Tottrup, H., Grubb, A., Abrahason, M., Olafsson, I. and Carlsen, S. (1989) Gene **79**, 325-332.

de Boer, H. A., Comstock, L. J. and Vasser, M. (1983) Proc. Natl. Acad. Sci. USA **80**, 21-25.

Denefle, P., Kovarik, S., Guitton, J.-D., Cartwright, T. und Mayaux, J.-F. (1987) Gene **56,** 61-70.

Deuel, T. F. and Huang, J. S. (1984) J. Clin. Invest. 74, 669-676.

Devare, S. G., Shatzman, A., Robbins, K. C., Rosenberg, M., Aaronson, S. A., (1984) Cell **36,** 43-49.

Duvoisin, R. M., Belin, D. und Krisch, H. M. (1986) Gene **45,** 193-201.

Eichner, W., Jäger, V., Herbst, D., Hauser, H. and Hoppe, J. (1989) Eur. J. Biochem. **185,** 135-140.

Ellis, J., Ozaki, L. S., Gwadz, R. W., Cochrane, A. H., Nussenzweig, V. and Gordon, G. N. (1983) Nature **302,** 536-538.

Emerick, A. W., Bertolani, B. L., Ben-Bassat, A., White, T. J. and Konrad M. W. (1984) Biotechnology **2,** 165-168.

Gentz, R., Kuys, Y., Zwieb, C., Taatjes, D., Taatjes, H., Bannwarth, W., Stueber, D. and Ibrahimi, I. (1988) J. Bacteriol. **170,** 2212-2220.

George, H. J., L'Italien, J. J., Pilacinski, W. P., Glassman, D. L. und Krzyzek, R. A: (1985) DNA **4,** 273-281.

Germino, J. and Bastia, D. (1984) Proc. Natl. Acad. Sci. USA **81,** 4692-4696.

Gill, J. A., Sumpter, J. P., Donaldson, E. M., Dye, H. M., Souza, L., Berg, T., Wypych, J. und Langley, K. (1985) Biotechnology **3,** 643-646.

Glick, B. R. and Whitney, G. K. (1987) J. Industrial Microbiol. **1,** 277-282.

Goeddel, D. V., Kleid, D. G., Bolivar, F., Heynecker, H. L., Yansura, D. G., Crea, R., Hirose, T., Kraszewski, A., Itakura, K. and Riggs, A. D. (1979) Proc. Natl. Acad. Sci. USA **76,** 106-110.

Gold, L. (1990) Methods Enzymol. **185,** 11-14.

Gray, G. L., Baldridge, J. S., McKeown, K. S., Heynecker, H. L. and Chang, C. N. (1985) Gene **39,** 247-254.

Greenhalgh, D. G., Sprugel, K. H., Murray, M. J. and Ross, R. (1990) Am. J. Pathol. **136,** 1235-1246.

Gross, G., Mielke, C., Hollatz, I., Blöcker, H. and Frank, R. (1990) J. Biol. Chem. **265,** 17627-17636.

Gross, M., Sweet, R. W., Sathe, G., Yokoyama, S., Fasano, O., Goldfarb, M., Wigler, M. und Rosenberg, M. (1985) Mol. Cel. Biol. **5,** 1015-1024.

Hammacher, A., Hellmann, U., Johnsson, A., Östman, A., Gunnarsson, K., Westermark, B., Wasteson, Å. and Heldin, C.-H. (1988) J. Biol. Chem. **263,** 16493-16499.

Hart, C. E., Forstrom, J. W., Kelly, J. D., Seifert, R. A., Smith, R. A., Ross, R., Murray, M. J. and Bowen-Pope, D. F. (1988) Science **240,** 1529-1531.

Hart, C. E., Bailey, M., Curtis, D. A., Osborn, S., Raines, E., Ross, R. and Forstrom, J. W. (1990) Biochemistry **29,** 166-172.

Heldin, C.-H., Bäckström, G., Östman, A., Hammacher, A., Rönnstrand, L., Rubin, K., Nister, M. and Westermark, B. (1988) EMBO J. **7,** 1387-1393.

Henning, U., Cole, S. T., Bremer, E., Hindennach, I. and Schaller, H. (1983) Eur. J. Biochem. **136,** 233-240.

Hoppe, J., Schumacher, L., Eichner, W. and Weich, H.A. (1987), FEBS Lett. **223,** 243-246.

Hoppe, J., Weich, H. A. and Eichner, W. (1989) Biochemistry **28,** 2956-2960.

Hoppe, J., Weich, H. A., Eichner, W. and Tatje, D. (1990) Eur. J. Biochem. **187,** 207-214.

Hosang, M., Rouge, M., Wipf, B., Eggiman, B., Kaufmann, F. and Hunziker, W. (1989) J. Cell. Physiol. **149,** 558-564.

Hsiung, H. M., Mayne, N. G. and Becker, G. W. (1986) Biotechnology **4,** 991-995.

Hsiung, H. M., Cantrell, A., Luirink, J., Oudega, B., Veros, A. J. and Becker, G. W. (1989) Biotechnology **7,** 267-271.

Ikehara, M., Ohtsuka, E., Tokunaga, T., Taniyama, Y., Iwai, S., Kitano, K., Miyamoto, S., Ohgi, T., Sakuragawa, Y., Fujiyama, K., Ikari, T., Kobayashi, M., Miyake, T., Shibahara, S., Ono, A., Ueda, T., Tanaka, T., Baba, H., Miki, T., Sakurai, A., Oishi, T., Chisaka, O. und Matsubara, K. (1984) Proc. Natl. Acad. Sci. USA **81,** 5956-5960.

Itakura, K., Hirose, T., Crea, R. and Riggs, A. D. (1977) Science **198,** 1056-1063.

Ito, K. (1981) Cell **24,** 707-717.

Ivanoff, L. A., Towatari, T., Ray, J., Korant, B. D. und Petteway, S. R. (1986) Proc. Natl. Acad. Sci. USA **83,** 5392-5396.

Jacob, C. O., Leitner, M., Salomon, D. and Arnon, R. (1985) EMBO J. **4,** 3339-3343.

Jay, G., Khoury, G., Seth, A. K. and Jay, E. (1981) Proc. Natl. Acad. Sci. USA **78,** 5543-5548.

Johnsson, A., Heldin, C.-H., Wasteson, A., Westermark, B., Deuel, T. F., Huang, J. S., Seeburg, P. H., Gray, A., Ullrich, A., Scrace, G., Stroobant, P. and Waterfield, M. D. (1984) EMBO J. **136,** 921-928.

Kane, J. F. and Hartley, D. L. (1988) TIBTECH **6,** 95-101.

Kato, C., Kobayashi, T., Kudo, T., Furusato, T., Murakami, Y., Tanaka, T., Baba, H., Oishi, T., Ohtsuka, E., Ikehara, M., Yanagida, T., Kato, H., Moriyama, S. and Horikoshi, K. (1987) Gene **54,** 197-202.

Kaytes, P. S., Theriault, N. Y., Poorman, R. A., Murakami, K. und Tomich, C.-S. C. (1986) J. Biotechnology **4,** 205-218.

Kelly, J. D., Raines, E. W., Ross, R. and Murray, M. J. (1985) EMBO J. **4,** 3399-3405.

Kishimoto, F., Gomi, H., Kanaoka, M., Nakatani, T., Ito, A., Katoh, T., Agui, H., Sumida, S. und Ogino, S. (1986) Gene **45,** 311-316.

Klein, B. K., Hill, S. R., Devine, C. S., Rowold, E., Smith, C. E., Galosy, S. and Olins, P. O. (1991) Biotechnology **9,** 869-872.

Kleid, D. G., Yansura, D., Small, B., Dowbenko, D., Moore, D. M., Grubman, M. J., McKercher, P. D., Morgan, D. O., Robertson, B. H. and Bachrach, H. L. (1981) Science, **214,** 1125-1128.

Kronheim, S. R., Cantrell, M. A., Deeley, M. C., March, C. J., Glackin, P. J., Anderson, D. M., Hemenway, T., Merriam, J. E., Cosman, D. und Hopp, T. P. (1986) Biotechnology **4**, 1078-1082.

Laemmli, U. K. (1970) Nature **227**, 680-685.

Langley, K. E., Lai, P.-H., Wypych, J., Everett, R. R., Berg, T. F., Krabill, L. F., Davis, J. M. und Souza, L. M. (1987A) Eur. J. Biochem. **163**, 323-330.

Langley, K. E., Berg, T. F., Strickland, T. W., Fenton, D. M., Boone, T. C. und Wypych, J. (1987B) Eur. J. Biochem. **163**, 313-321.

Lee, J. M. and Ullrich, A. (1984) Eur. Pat. Appl. 0128733.

Lee, N., Zhang, S.-Q., Cozzitorto, J., Yang, J.-S. and Testa, D. (1987) Gene **58**, 77-86.

Levi, S., Cesarini, G., Arosio, P., Lorenzetti, R., Soria, M., Sollazo, M., Albertini, A. und Cortese, R. (1987) Gene **51**, 269-274.

Lord, S. (1985) DNA **4,** 33-38.

Mandecki, W., Powell, B. S., Mollison, K. W., Carter, G. W. und Fox, J. L. (1986) Gene **43,** 131-138.

Maniatis, T., Fritsch, E. F., and Sambrook, J. (1982) *Molecular Cloning. A Laboratory Manual,* Cold Spring Harbour Laboratory, Cold Spring Harbour, NY.

Marston, F. A. O. (1986) Biochem. J. **240,** 1-12.

Marston, F. A. O., Lowe, P. A., Doel, M. T., Schoemaker, J. M., White, S. und Angal, S. (1984) Biotechnology **2,** 800-804.

Martial, J. A., Hallewell, R. A., Baxter, J. D. and Goodman, H. M. (1979) Science **205,** 602-606.

Matoskova, B., Rorsman, F., Svensson, V. and Betsholtz, C. (1989), Mol. Cell. Biol. **9**, 3148-3150.

McCarthy, J. E. G. (1991) Adv. Gene Technol. **2**, 145-175.

McCarthy, J. E. G. and Gualerzi, C. (1990) Trends Genet. **6**, 78-85.

McCarthy, J. E. G., Schairer, H. U. und Sebald, W. (1985) EMBO J. **4,** 519-526.

Meyers, C. A., Johanson, K. O., Miles, L. M., McDevitt, P. J., Simon, P. L., Webb, R. L., Chen, M.-J., Holskin, B. P., Lillquist, J. S. und Young, P. R. (1987) J. Biol. Chem. **262**, 11176-11181.

Miki, T., Yasukochi, T., Nagatani, H., Furuno, M., Orita, T., Yamada, H., Imoto, T. und Horiuchi, T. (1987) Protein Engineering 1, 327-332.

Moks, T., Abrahmsen, L., Österlöf, B., Josephson, S., Östling, M., Enfors, S.-O., Persson, I., Nilsson, B. and Uhlen, M. (1987) Bio/Technology **5**, 379-382.

Mustoe, T. A., Purdy, J., Gramates, P., Deuel, T. F., Thomason, A. and Pierce, G. F. (1989) Am. J. Surg. **158**, 345-350.

Nagahari, K., Kanaya, S., Munakata, K., Aoyagi, Y. and Mizushima, S. (1985) EMBO J. **4**, 3589-3592.

Nagai, K. and Thogersen, H. C. (1984) Nature **309**, 810-812.

Nakamura, K. and Inouye, M. (1982) EMBO J. **6**, 771-775.

Nambiar, K. P., Stackhouse, J., Presnell, S. R. and Benner, S. A. (1987) Eur. J. Biochem. **163**, 67-71.

Nicaud, J.-M., Mackman, N. and Holland, I. B. (1986) J. Biotechnol. **3**, 255-270.

Nilsson, B. and Abrahmsen, L. (1990) Methods Enzymol. **185**, 144-161.

Nister, M., Hammacher, A., Mellström, K., Siegbahn, A., Rönnstrang, L., Westermark, B. and Heldin, C.-H. (1988), Cell **52**, 791-799.

Obukowicz, M. G., Turner, M. A., Wong, E. Y. and Tacon, W. C. (1988) Mol. Gen. Genet. **215**, 19-25.

Östman, A., Rall, L., Hammacher, A., Wormstead, M. A., Coit, D., Valenzuela, P., Betsholtz, C., Westermark, B. and Heldin, C.-H. (1988) J. Biol. Chem. **263**, 16202-16208.

Ohsuye, K., Nomura, M., Tanaka, S., Kubota, I., Nakazato, H., Shinagawa, H., Nakata, A. and Noguchi, T. (1983) Nucleic Acids Res. **11**, 1283-1294.

Oka, T., Sakamoto, S., Miyoshi, K.-I., Fuwa, T., Yoda, K., Yamasaki, M., Tamura, G. and Miyake, T. (1985) Proc. Natl. Acad. Sci. USA **82**, 7212-7216.

Olins, P. O. and Rangwala, S. H. (1990) Methods Enzymol. **185**, 115-119.

Pennica, D., Hayflick, J. S., Bringman, T. S., Palladino, M. A. und Goeddel, D. V. (1985) Proc. Natl. Acad. Sci. USA **82**, 6060-6064.

Peters, M. A., Lau, E. P., Snitman, D. L., van Wyk, J. J., Underwood, L. E., Russell, W. E. and Svoboda, M. E. (1985) Gene **35**, 83-89.

Pierce, G. F., Mustoe, T. A., Lingelbach, J., Masakowski, V. R., Griffin, G., Senior, R. M. and Deuel, T. F. (1989) J. Cell. Biol. 109, 429-440.

Pierce, G. F., VandeBerg, J., Rudolph, R., Tarpley, J. and Mustoe, T. A. (1991A) Am. J. Pathol. **138**, 629-646.

Pierce, G. F., Brown, D. and Mustoe, T. A. (1991B) J. Lab. Clin. Med. **117**, 373-382.

Pollitt, S. and Zalkin, H. (1983) J. Bacteriol. **153**, 27-32.

Potts, J. T., Kronenberg, H. M. and Rosenblatt, M. (1982) Adv. Protein Chem. **35**, 323-398.

Ratner, L., Josephs, S. F., Jarrett, R., Reitz, M. S. and Wong-Staal, F. (1985), Nucl. Acids Res. **13**, 5007-5018.

Reilly, C. F. and Broski, J. E. (1989) Biochem. Biophys. Res. Commun. **160**, 1047-1054.

Remaut, E., Stanssens, P. and Fiers, W. (1981) Gene **15**, 81-93.

Remaut, E., Stanssens, P. and Fiers, W. (1983) Nucleic Acids Res. **11**, 4677-4688.

Robson, M. C., Phillips, L. G., Thomason, A., Altrock, B. W., Pence, P. C., Heggers, J. P., Johnston, A. F., McHugh, T. P., Anthony, M. S. Robson, L. E., Odom. L. L., Yanagihara, D. and Pierce, G. F. (1992) Ann. Plast. Surg. **29**, 193-201.

Ross R., Glomset, J. A., Kariya, B. and Harker, L. (1974) Proc. Natl. Acad. Sci. U.S.A. **71**, 1207-1210.

Sachinidis, A., Locher, R., Vetter, W., Tatje, D. and Hoppe, J. (1990A) J. Biol. Chem. **265**, 10238-10243.

Sachinidis, A., Locher, R., Hoppe, J. and Vetter, W. (1990B) FEBS Lett. **275,** 95-98.

Saito, Y., Ishii, Y., Niwa, M. and Ueda, I. (1987) J. Biochem. **101,** 1281-1288.

Sambrook, J., Fritsch, E. F. und Maniatis, T. (1989) *Molecular Cloning. A Laboratory Manual,* Cold Spring Harbour Laboratory Press.

Sawyer, J. R. and Blattner, F. R. (1991) Protein Engineering **4**, 947-953.

Schauder, B., Blöcker, H., Frank, R. and McCarthy, J. E. G. (1987) Gene **52**, 279-283.

Schauder, B. and McCarthy, J. E. G. (1989) Gene **78**, 59-72.

Schein, C. H. (1989) Biotechnology **7**, 1141-1149.

Scherer, G. F. E., Walkinshaw, M. D., Arnott, S. and Morre, D. J. (1980) Nucleic Acids Res. **8**, 3895-3907.

Schoner, B. E., Hsiung, H. M., Belagaje, R. M., Mayne, N. G. and Schoner, R. G. (1984) Proc. Natl. Acad. Sci. U.S.A. **81**, 5403-5407.

Seeburg, P. H., Shine, J., Martial, J. A., Ivarie, R. D., Morris, J. A., Ullrich, A., Baxter, J. D. and Goodman, H. M. (1978) Nature **276**, 795-798.

Shine, J., Fettes, I., Lan, N. C. Y., Roberts, J. L. and Baxter, J. D. (1980) Nature **285**, 456-461.

Shipley, G. D., Childes, C. B., Volkenant, M. E. and Moses, H. L. (1984) Cancer Res. **44**, 710-716.

Siegbahn, A., Hammacher, A., Westermark, B. and Heldin, C.-H. (1990) J. Clin. Invest. **85**, 916-920.

Simon, L. D., Randolph, B., Irwin, N. und Binkowski, G. (1983) Proc. Natl. Acad. Sci. USA **80**, 2059-2062.

Simons, G., Remaut, E., Allet, B., Devos, R. und Fiers, W. (1984) Gene **28,** 55-64.

Sollazzo, M., Frank, R. and Cesareni, G. (1985) Gene **37,** 199-206.

Spanjaard, R. A., van Dijk, M. C. M., Turion, A. J. and van Duin, J. (1989) Gene **80,** 345-351.

Stader, J. A. and Silhavy, T. J. (1990) Methods Enzymol. **185,** 166-187.

Stanley, K. K. and Luzio, J. P. (1984) EMBO J. **3**, 1429-1434.

Stanssens, G., Remaut, E. and Fiers, W. (1985) Gene **36**, 211-223.

Stark, M. J. R. (1987) Gene **54**, 255-267.

Strauch, K. L. and Beckwith, J. (1988) Proc. Natl. Acad. Sci. USA **85,** 1576-1580.

Studier, F. W. and Moffatt, B. A. (1986) J. Mol. Biol. **189**, 113-130

Studier, F. W., Rosenberg, A. H., Dunn, J. J. and Dubendorf, J. W. (1990) Methods Enzymol. **185,** 60-89.

Swamy, K. H. S. and Goldberg, A. L. (1982) J. Bacteriol. **149,** 1027-1033.

Szoka, P. R., Schreiber, A. B., Chan, H. and Murthy, J. (1986) DNA **5**, 11-20.

Tabor, S. and Richardson, C. C. (1985) Proc. Natl. Acad. Sci. **82**, 1074-1078.

Takagi, H., Morinaga, Y., Tsuchia, M., Ikemura, H. and Inouye, M. (1988) Biotechnology **6**, 948-950.

Takahara, M., Sagai, H., Inouye, S. and Inouye, M. (1988) Biotechnology, **6**, 195-198.

Talmadge, K., Kaufman, J. and Gilbert, W. (1980) Proc. Natl. Acad. Sci. USA **77**, 3988-3992.

Talmadge, K. and Gilbert, W. (1982) Proc. Natl. Acad. Sci. USA **79**, 1830-1833.

Taniguchi, T., Guarente, L., Roberts, T. M., Kimelman, D., Douhan III, J. und Ptashne, M. (1980) Proc. Natl. Acad. Sci. USA **77**, 5230-5233.

Tanizawa, Y., Kishi, F., Kaneko, T. und Nakazawa, A. (1987) J. Biochem. **101**, 1289-1296.

Tsuji, T., Nakagawa, R., Sugimoto, N. und Fukuhara, K.-I. (1987) Biochemistry **26**, 3129-3134.

Tuggle, C. K. und Fuchs, J. A. (1985) J. Bacteriol. **162**, 448-450.

Uhlen, M. and Moks, T. (1990) Methods Enzymol. **185**, 129-143.

Ullman, A. (1984) Gene, **29**, 27-31.

van der Werf, S., Dreano, M., Bruneau, P., Kopecka, H. and Girard, M. (1983) Gene **23**, 85-93.

Varadarajan, R., Szabo, A. and Boxer, S. G. (1985) Proc. Natl. Sci. USA **82**, 5681-5684.

Villa-Komaroff, L., Efstratiadis, A., Broome, S., Lomedico, P., Tizard, R., Naber, S. P., Chick, W. L., Gilbert, W. (1978) Proc. Natl. Acad. Sci. USA **75**, 3727-3731.

Wang, J. Y. J. and Williams, L. T. (1984) J. Biol. Chem. **259**, 10645-10648.

Weich, H. A., Sebald, W., Schairer, H. U. and Hoppe, J. (1986), FEBS Lett. **198**, 344-348.

Wingender, E., Bercz, G., Blöcker, H., Frank, R. and Mayer, H. (1989) J. Biol. Chem. **264**, 4367-4373.

Winkler, M. E., Blaber, M., Bennett, G. L., Holmes, W. und Vehar, G. A. (1985) Biotechnology **3**, 990-998.

Wise, R. J., Orkin, S. H. and Collins, T. (1989) Nucl. Acids Res. **17**, 6591-6601.

Wong, E. Y., Seetharam, R., Kotts, C. E., Heeren, R. A., Klein, B. K., Braford, S. R., Mathis, K. J., Bishop, B. F., Siegel, N. R., Smith, C. E. and Tacon, W. C. (1988) Gene **68**, 193-203.

Yanisch-Perron, C., Vieira, J. and Messing, J. (1985) Gene **33**, 103-119.

Yansura, D. G. (1990) Methods Enzymol. **185**, 161-166.

Yansura, D. G. and Henner, D. J. (1990) Methods Enzymol. **185**, 54-60.

Young, R. M., Mendoza, A. E., Collins, T. and Orkin, S. H. (1990) Mol. Cell. Biol. **10**, 6051-6054.

Zemel-Dreasen, O. und Zamir, A. (1984) Gene **27**, 315-322.

Zuker, M. und Stiegler, P. (1981) Nucleic Acids Res. **9**, 133-148

Zurawski, S. M., Mosmann, T. R., Benedik, M. und Zurawski, G. (1986) J. Immunology **137**, 3354-3360.

**Abkürzungen:**

bp - Basenpaar(e)
BSA - Rinderserumalbumin
C - Cistron
EDTA - Ethylendiamintetraessigsäure
ELISA - enzyme-linked immunosorbent assay
IgG - Immunglobulin der Klasse G
nt - Nukleotid(e)
p - Promotor
PBS - phosphatgepufferte Kochsalzlösung
PDGF - Wachstumsfaktor aus Thrombozyten
r - Gen für ein Repressormolekül
t - Transkriptions-Terminator
TIR - Translations-Initiations Region
TIS - Translation-Initiations Sequenz

Patentansprüche

1. Verfahren zur rekombinanten Herstellung multimerer Proteine in Bakterienzellen, dadurch gekennzeichnet, daß man

   a) die Zellen mit einem Vektor transformiert, der eine multicistronische Expressionseinheit der allgemeinen Formel

$$p - (TIS - C)_n$$

für die simultane und direkte Expression der Komponenten und/oder Untereinheiten eines multimeren Proteins in ein und derselben Bakterienzelle enthält, in der

"p" ein bakterieller transkriptionaler Promotor und

"(TIS - C)$_n$" repetitive Gruppen sind, innerhalb derer

"C" jeweils für eine Untereinheit cder eine Komponente eines multimeren Proteins kodierende DNA-Sequenzen sind, die keine für ein Signalpeptid kodierende Region umfassen, wobei die Sequenzen "C" der aufeinander folgenden Gruppen "TIS - C" untereinander gleich und/oder verschieden sein können,

"TIS" synthetische und/oder natürliche bakterielle nicht translatierte "leader" Sequenzen sind, die jeweils mit "C" eine chimäre "TIR" bilden, wobei die Sequenzen "TIS" der aufeinander folgenden Gruppen "TIS - C" untereinander gleich und/oder verschieden sein können, und

n eine natürliche Zahl $\geq 2$ ist,

wobei "p", "TIS" und "C" jeweils operativ miteinander verknüpft sind und die Expressionsrate der Protein-kodierenden Sequenzen "C" durch Auswahl der jeweils zugehörigen Sequenz "TIS" den stöchiometrischen Verhältnissen gemäß eingestellt wird, die für die nachfolgende Renaturierung und/oder Rekonstitution notwendig ist,

b) die transformierten Zellen in einem geeigneten Nährmedium kultiviert und anschließend

c) die Zellen von dem Medium abtrennt, die koexprimierten Expressionsprodukte der Sequenzen "C" aus dem Cytoplasma der Zellen isoliert, gegebenenfalls renaturiert und zu dem funktionellen multimeren Protein rekonstituiert.

2. Multicistronische Expressionseinheit zur Verwendung in dem Verfahren nach Anspruch 1, gekennzeichnet durch die allgemeine Formel

$$p - (TIS - C)_n,$$

in der

"p" ein bakterieller transkriptionaler Promotor und

"(TIS - C)$_n$" repetitive Gruppen sind, innerhalb derer

"C" jeweils für eine Untereinheit oder eine Komponente eines multimeren Proteins kodierende DNA-Sequenzen sind, die keine für ein Signalpeptid kodierende Region umfassen, wobei die Sequenzen "C" der aufeinander folgenden Gruppen "TIS - C" untereinander gleich und/oder verschieden sein können,

"TIS" synthetische und/oder natürliche bakterielle nicht translatierte "leader" Sequenzen sind, die jeweils mit "C" eine chimäre "TIR" bilden, wobei die Sequenzen "TIS" der aufeinander folgenden Gruppen "TIS - C" untereinander gleich und/oder verschieden sein können, und

n eine natürliche Zahl $\geq 2$ ist,

wobei "p", "TIS" und "C" jeweils operativ miteinander verknüpft sind und die Sequenz "TIS" so ausgewählt wird, daß die Expressionsrate der Protein-kodierenden Sequenzen "C" den stöchiometrischen Verhältnissen gemäß eingestellt wird, die für die nachfolgende Renaturierung und/oder Rekonstitution notwendig ist.

3. Multicistronische Expressionseinheit nach Anspruch 2, **dadurch gekennzeichnet**, daß "n" 2 oder 3 ist.

4. Multicistronische Expressionseinheit nach den Ansprüchen 2 oder 3, **dadurch gekennzeichnet**, daß sie an ihrem 3' Ende operativ mit einem bakteriellen Transkriptions-Terminator verknüpft ist.

5. Multicistronische Expressionseinheit nach den Ansprüchen 2 bis 4, **dadurch gekennzeichnet**, daß sie operativ an ein Gen "r" mit eigenem Promotor gekoppelt ist, welches für ein Repressormolekül für den Promotor "p" kodiert.

6. Multicistronische Expressionseinheit nach Anspruch 5, **dadurch gekennzeichnet**, daß "p" der $\lambda P_R$-Promotor oder der $\lambda P_L$-Promotor und "r" das cl857-Gen für den temperatursensitiven $\lambda$-Repressor ist.

7. Multicistronische Expressionseinheit nach den Ansprüchen 1 bis 6, **dadurch gekennzeichnet**, daß "TIS" die Sequenz TIS B gemäß SEQ. ID. NO: 15 und/oder TIS E gemäß SEQ. ID. NO: 14 ist.

8. Multicistronische Expressioneinheit nach den Ansprüchen 1 bis 7, **dadurch gekennzeichnet**, daß die Sequenzen "C" für die verschiedenen Komponenten oder Untereinheiten von VEGF, Scatter-Faktor (HGF-SF), Mitgliedern der TGF-β-Superfamilie, Bone Morphogenic Protein (BMP), Faktoren der Integrin/Cadherin Familie, Histokampatibilitätsantigenen, Hämoglobin, T-Zellrezeptoren oder des AB-Heterodimeren des Wachstumsfaktors aus Blutplättchen (PDGF) oder deren natürliche oder synthetische Varianten kodieren.

9. Multicistronische Expressionseinheit nach den Ansprüchen 1 bis 8, **dadurch gekennzeichnet**, daß "n" 2 ist und die Sequenzen "C" der beiden repetitiven Einheiten "(TIS - C)$_2$" alternativ ein für die A- oder die B-Kette von PDGF oder ein biologisch aktives Analogon oder ein Fragment derselben kodieren, wobei beide Sequenzen gleichzeitig in der Expressionseinheit vertreten sind.

10. Multicistronische Expressionseinheit nach Anspruch 9, **dadurch gekennzeichnet**, daß eine der Sequenzen "C" die PDGF-A$_K$ Sequenz (SEQ. ID. NO:12) oder die PDGF-A$_L$ Sequenz ist, welche für die reife PDGF-A-Kette kodiert.

11. Multicistronische Expressionseinheit nach den Ansprüchen 9 oder 10, **dadurch gekennzeichnet**, daß die jeweils andere Sequenz "C" die vollständige PDGF-B Sequenz (SEQ. ID. NO: 10) ist, welche für die reife PDGF-B-Kette (SEQ. ID. NO: 11) kodiert, oder das v-sis-Gen aus Simian Sarcoma Virus oder Varianten dieser Sequenzen ist.

12. Vektor zur Verwendung in dem Verfahren nach Anspruch 1, welcher eine multicistronische Expressionseinheit nach den Ansprüchen 2 bis 11 enthält.

13. Wirtszelle zur Verwendung in dem Verfahren nach Anspruch 1, welche eine Bakterienzelle transformiert mit einem Vektor nach Anspruch 12 ist.

14. Wirtszelle nach Anspruch 13, **dadurch gekennzeichnet**, daß sie eine *E. coli*-Zelle ist.

15. Expressionseinheit nach Anspruch 2, **dadurch gekennzeichnet**, daß es sich um eine Expressionseinheit für die simultane und direkte Expression für PDGF-A und PDGF-B kodierender DNA-Sequenzen in einer Bakterienzelle handelt, die die allgemeine Formel

$$p - TIS_1 - C_1 - TIS_2 - C_2 - t - r,$$

aufweist, in der

"p" der $\lambda P_R$-Promotor ist,

"C$_1$" und "C$_2$" alternativ Sequenzen enthalten, die für PDGF-A oder PDGF-B kodieren und beide Gene gleichzeitig in der Expressionseinheit enthalten sind,

TIS$_1$ und TIS$_2$ jeweils die Sequenz TIS B (SEQ. ID. NO: 15) oder TIS E (SEQ. ID. NO: 14) sind und untereinander gleich oder verschieden sein können,

"t" der Terminator des Bakteriophagen fd ist und

"r" das cl857-Gen für den temperatursensitiven $\lambda$-Repressor ist.

**16.** Expressionseinheit nach Anspruch 15, **dadurch gekennzeichnet**, daß $C_1$ und $C_2$ alternativ die PDGF-A-Sequenz gemäß SEQ. ID. NO: 12 oder die PDGF-B-Sequenz gemäß SEQ. ID. NO: 10 enthalten und beide Gene gleichzeitig in der Expressionseinheit enthalten sind.

**17.** Expressionseinheit nach Anspruch 16, **dadurch gekennzeichnet**, daß "$TIS_1$" die TIS B Sequenz gemäß SEQ. ID. NO: 15, "$TIS_2$" die TIS E Sequenz gemäß SEQ. ID. NO: 14, "$C_1$" die PDGF-B-Sequenz gemäß SEQ. ID. NO: 10 und "$C_2$" die PDGF-A-Sequenz gemäß SEQ . ID. NO: 12 sind.

**18.** Expressionseinheit nach Anspruch 16, **dadurch gekennzeichnet**, daß "$TIS_1$" und "$TIS_2$" die TIS B Sequenzen gemäß SEQ. ID. NO: 15, "$C_1$" die PDGF-B-Sequenz gemäß SEQ. ID. NO: 10 und "$C_2$" die PDGF-A-Sequenz gemäß SEQ. ID. NO: 12 sind.

**19.** Expressionseinheit nach Anspruch 16, **dadurch gekennzeichnet**, daß "$TIS_1$" und "$TIS_2$" die TIS B Sequenzen gemäß SEQ. ID. NO: 15 sind, "$C_1$" die PDGF-A-Sequenz gemäß SEQ. ID. NO: 12 und "$C_2$" die PDGF-B-Sequenz gemäß SEQ. ID. NO: 10 sind.

**20.** Rekombinanter Vektor zur Expression in Bakterienzellen, welcher eine bicistronische Expressionseinheit nach den Ansprüchen 15 bis 19 enthält.

**21.** Wirtszelle, welche eine Bakterienzelle transformiert mit einem Vektor nach Anspruch 20 ist.

**22.** Wirtszelle nach Anspruch 21, **dadurch gekennzeichnet**, daß sie eine *E. coli*-Zelle ist.

**23.** Wirtszelle nach Anspruch 22, **dadurch gekennzeichnet**, daß sie eine *E. coli*-Zelle ist, welche von den Klonen TG2/pBS/ PDGF-BA2 (Hinterlegungsnummer DSM 8336), TG2/pBS/PDGF-BA4 (Hinterlegungsnummer DSM 8338) oder TG2/pBS/PDGF-AB4 (Hinterlegungsnummer DSM 8337) abstammt.

**24.** Verfahren zur rekombinanten Herstellung von PDGF-AB in Bakterienzellen, **dadurch gekennzeichnet**, daß man Wirtszellen nach den Ansprüchen 21 bis 23 in einem geeigneten Medium kultiviert, anschließend die Zellen von dem Medium abtrennt, die Proteinkomponenten PDGF-A und PDGF-B aus dem Cytoplasma der Zellen isoliert, renaturiert und zu funktionellem PDGF-AB rekonstituiert.

**25.** Verfahren zur Herstellung von multimere Proteine enthaltenden pharmazeutischen oder kosmetischen Präparaten, **dadurch gekennzeichnet**, daß man die multimeren Proteine nach den Schritten a) bis c) des verfahrens nach Anspruch 1 herstellt und anschließend in Schritt d) die multimeren Proteine zusammen mit üblichen Hilfs- und Trägerstoffen zu pharmazeutischen oder kosmetichen Präparaten formuliert.

**26.** Verfahren zur Herstellung von PDGF-AB enthaltenden pharmazeutischen oder kosmetischen Präparaten, **dadurch gekennzeichnet**, daß man PDGF-AB nach dem Verfahren nach Anspruch 24 herstellt und anschließend zusammen mit üblichen Hilfs- und Trägerstoffen zu pharmazeutischen oder kosmetischen Präparaten formuliert.

**27.** Verfahren nach den Ansprüchen 25 oder 26, **dadurch gekennzeichnet**, daß das pharmazeutische Präparat eine Salbe, ein Spray, ein Gel, ein Wundverband, ein Pflaster oder eine Wundauflage ist.

**Claims**

**1.** Process for the recombinant preparation of multimeric proteins in bacterial cells, characterized in that

   a) the cells are transformed with a vector which contains a multicistronic expression unit of the general formula

$$p - (TIS - C)_n$$

   for the simultaneous and direct expression of the components and/or subunits of a multimeric protein in one and the same bacterial cell, in which

      "p" is a bacterial transcriptional promoter and

"(TIS - C)$_n$" are repetitive groups, within which
"C" are in each case DNA sequences which encode a subunit or a component of a multimeric protein, which sequences do not encompass any region encoding a signal peptide, where the "C" sequences of the consecutive "TIS - C" groups can be the same as and/or different from each other,
"TIS" are synthetic and/or natural bacterial, non-translated "leader" sequences which in each case form a chimeric "TIR" with "C", where the "TIS" sequences of the consecutive "TIS - C" groups can be the same as and/or different from each other, and
n is a natural number $\geq 2$,

where "p", "TIS" and "C" are in each case linked operatively to each other and the rate of expression of the protein-encoding "C" sequences is adjusted according to the stoichiometric ratios which are necessary for the subsequent renaturation and/or reconstitution by selecting the respective affiliated "TIS" sequence,
b) the transformed cells are cultivated in a suitable nutrient medium and subsequently
c) the cells are separated off from the medium, and the coexpressed expression products of the "C" sequences are isolated from the cytoplasm of the cells, optionally renatured and reconstituted to form the functional multimeric protein.

2. Multicistronic expression unit for use in the process according to Claim 1, characterized by the general formula

$$p - (TIS - C)_n,$$

in which

"p" is a bacterial transcriptional promoter and
"(TIS - C)$_n$" are repetitive groups, within which
"C" are in each case DNA sequences which encode a subunit or a component of a multimeric protein, which sequences do not encompass any region encoding a signal peptide, where the "C" sequences of the consecutive "TIS - C" groups can be the same as and/or different from each other,
"TIS" are synthetic and/or natural bacterial, non-translated "leader" sequences which in each case form a chimeric "TIR" with "C", where the "TIS" sequences of the consecutive "TIS - C" groups can be the same as and/or different from each other, and
n is a natural number $\geq 2$,

where "p", "TIS" and "C" are in each case linked operatively to each other and the "TIS" sequence is selected such that the rate of expression of the protein-encoding "C" sequences is adjusted according to the stoichiometric ratios which are necessary for the subsequent renaturation and/or reconstitution.

3. Multicistronic expression unit according to Claim 2, characterized in that "n" is 2 or 3.

4. Multicistronic expression unit according to Claims 2 or 3, characterized in that it is linked operatively at its 3' end to a bacterial transcription terminator.

5. Multicistronic expression unit according to Claims 2 to 4, characterized in that it is operatively coupled by its own promoter to a gene "r" which encodes a repressor molecule for the promoter "p".

6. Multicistronic expression unit according to Claim 5, characterized in that "p" is the $\lambda P_R$ promoter or the $\lambda P_L$ promoter and "r" is the cl857 gene for the temperature-sensitive $\lambda$ repressor.

7. Multicistronic expression unit according to Claims 1 to 6, characterized in that "TIS" is the sequence TIS B according to SEQ ID NO: 15 and/or TIS E according to SEQ ID NO: 14.

8. Multicistronic expression unit according to Claims 1 to 7, characterized in that the "C" sequences encode the various components or subunits of VEGF, scatter factor (HGF-SF), members of the TGF-$\beta$ super family, bone morphogenic protein (BMP), factors of the integrin/cadherin family, histocompatibility antigens, haemoglobin, T-cell receptors, or the AB heterodimer of platelet-derived growth factor (PDGF), or their natural or synthetic variants.

9. Multicistronic expression unit according to Claims 1 to 8, characterized in that "n" is 2 and the "C" sequences of the two repetitive "(TIS - C)$_2$" units alternatively encode the A chain or the B chain of PDGF, or a biologically active analogue or a fragment thereof, both sequences simultaneously being represented in the expression unit.

10. Multicistronic expression unit according to Claim 9, characterized in that one of the "C" sequences is the PDGF-A$_K$ sequence (SEQ ID NO: 12) or the PDGF-A$_L$ sequence which encodes the mature PDGF-A chain.

11. Multicistronic expression unit according to Claims 9 or 10, characterized in that the respective other "C" sequence is the complete PDGF-B sequence (SEQ ID NO: 10) which encodes the mature PDGF-B chain (SEQ ID NO: 11) or the v-sis gene from simian sarcoma virus, or variants of these sequences.

12. Vector for use in the process according to Claim 1, which vector contains a multicistronic expression unit according to Claims 2 to 11.

13. Host cell for use in the process according to Claim 1, which host cell is a bacterial cell transformed with a vector according to Claim 12.

14. Host cell according to Claim 13, characterized in that it is an *E. coli* cell.

15. Expression unit according to Claim 2, characterized in that it is an expression unit for the simultaneous and direct expression in a bacterial cell of DNA sequences encoding PDGF-A and PDGF-B, which expression unit has the general formula

$$p - TIS_1 - C_1 - TIS_2 - C_2 - t - r,$$

in which

"p" is the $\lambda P_R$ promoter,
"C$_1$" and "C$_2$" alternatively contain sequences which encode PDGF-A or PDGF-B and both genes are simultaneously contained in the expression unit,
TIS$_1$ and TIS$_2$ are in each case the sequence TIS B (SEQ ID NO: 15) or TIS E (SEQ ID NO: 14) and can be the same as or different from each other,
"t" is the terminator of the bacteriophage fd, and
"r" is the cI857 gene for the temperature-sensitive $\lambda$ repressor.

16. Expression unit according to Claim 15, characterized in that C$_1$ and C$_2$ alternatively contain the PDGF-A sequence according to SEQ ID NO: 12 or the PDGF-B sequence according to SEQ ID NO: 10 and both genes are simultaneously contained in the expression unit.

17. Expression unit according to Claim 16, characterized in that "TIS$_1$" is the TIS B sequence according to SEQ ID NO: 15, "TIS$_2$" is the TIS E sequence according to SEQ ID NO: 14, "C$_1$" is the PDGF-B sequence according to SEQ ID NO: 10 and "C$_2$" is the PDGF-A sequence according to SEQ ID NO: 12.

18. Expression unit according to Claim 16, characterized in that "TIS$_1$" and "TIS$_2$" are the TIS B sequences according to SEQ ID NO: 15, "C$_1$" is the PDGF-B sequence according to SEQ ID NO: 10 and "C$_2$" is the PDGF-A sequence according to SEQ ID NO: 12.

19. Expression unit according to Claim 16, characterized in that "TIS$_1$" and "TIS$_2$" are the TIS B sequences according to SEQ ID NO: 15, "C$_1$" is the PDGF-A sequence according to SEQ ID NO: 12 and "C$_2$" is the PDGF-B sequence according to SEQ ID NO: 10.

20. Recombinant vector for expression in bacterial cells, which vector contains a bicistronic expression unit according to Claims 15 to 19.

21. Host cell which is a bacterial cell transformed with a vector according to Claim 20.

22. Host cell according to Claim 21, characterized in that it is an *E. coli* cell.

**23.** Host cell according to Claim 22, characterized in that it is an *E. coli* cell which is derived from clones TG2/pBS/PDGF-BA2 (deposition number DSM 8336), TG2/pBS/PDGF-BA4 (deposition number DSM 8338) or TG2/pBS/PDGF-AB4 (deposition number DSM 8337).

**24.** Process for the recombinant preparation of PDGF-AB in bacterial cells, characterized in that host cells according to Claims 21 to 23 are cultivated in a suitable medium, the cells are subsequently separated off from the medium, and the protein components PDGF-A and PDGF-B are isolated from the cytoplasm of the cells and renatured, and reconstituted to form functional PDGF-AB.

**25.** Process for the production of pharmaceutical or cosmetic preparations containing multimeric proteins, characterized in that the multimeric proteins are prepared according to steps a) to c) of the process according to Claim 1 and subsequently in step d) the multimeric proteins are formulated together with customary auxiliary agents and excipients to form pharmaceutical or cosmetic preparations.

**26.** Process for the production of pharmaceutical or cosmetic preparations containing PDGF-AB, characterized in that PDGF-AB is prepared by the process according to Claim 24 and subsequently formulated together with customary auxiliary agents and excipients to form pharmaceutical or cosmetic preparations.

**27.** Process according to Claims 25 or 26, characterized in that the pharmaceutical preparation is an ointment, a spray, a gel, a wound bandage, a plaster or a wound dressing.

**Revendications**

**1.** Procédé pour la production par recombinaison de protéines multimères dans des cellules bactériennes, caractérisé en ce que

a) les cellules sont transformées par un vecteur qui contient une unité d'expression multicistronique de formule générale

$$p - (TIS - C)_n$$

pour l'expression directe et simultanée des composants et/ou sous-unités d'une protéine multimère dans une seule et même cellule bactérienne,
formule dans laquelle

"p" représente un promoteur transcriptionnel bactérien et
"(TIS - C)$_n$ sont des groupes répétitifs, à l'intérieur desquels

"C"       sont chaque fois des séquences d'ADN codant pour une sous-unité ou un composant d'une protéine multimère, qui ne comprennent pas de région codant pour un peptide signal, les séquences "C" des groupes "TIS - C" successifs pouvant être identiques et/ou différentes les unes des autres,
"TIS"     sont des séquences "de tête" non traduites, synthétiques et/ou bactériennes naturelles, qui forment chacune avec "C" une "TIR" (séquence d'initiation de traduction) chimère, les séquences "TIS" des groupes "TIS - C" successifs pouvant être identiques et/ou différentes les unes des autres, et
n         est un nombre entier $\geq 2$,

p, "TIS" et "C" étant fonctionnellement liés entre eux et le taux d'expression des séquences "C" codant pour la protéine étant ajusté selon les rapports stoechiométriques, par le choix de la séquence "TIS" correspondante dans chaque cas, qui est requise pour la reconstitution et/ou la renaturation subséquentes,

b) les cellules transformées sont cultivées dans un milieu nutritif approprié et ensuite
c) les cellules sont séparées du milieu, les produits d'expression des séquences "C", exprimés simultanément, sont isolés à partir du cytoplasme des cellules, éventuellement renaturés et reconstitués en la protéine multimère fonctionnelle.

2. Unité d'expression multicistronique pour utilisation dans le procédé selon la revendication 1, caractérisée par la formule générale

$$p - (TIS - C)_n$$

dans laquelle

"p" représente un promoteur transcriptionnel bactérien et
"$(TIS - C)_n$ sont des groupes répétitifs, à l'intérieur desquels

"C" sont chaque fois des séquences d'ADN codant pour une sous-unité ou un composant d'une protéine multimère, qui ne comprennent pas de région codant pour un peptide signal, les séquences "C" des groupes "TIS - C" successifs pouvant être identiques et/ou différentes les unes des autres,

"TIS" sont des séquences "de tête" non traduites, synthétiques et/ou bactériennes naturelles, qui forment chacune avec "C" une "TIR" chimère, les séquences "TIS" des groupes "TIS - C" successifs pouvant être identiques et/ou différentes les unes des autres, et

n est un nombre entier $\geq 2$,

p, "TIS" et "C" étant fonctionnellement liés entre eux et la séquence "TIS" qui est requise pour la reconstitution et/ou la renaturation subséquentes étant choisie de manière que le taux d'expression des séquences "C" codant pour la protéine soit ajusté selon les rapports stoechiométriques.

3. Unité d'expression multicistronique selon la revendication 2, caractérisée en ce que "n" est 2 ou 3.

4. Unité d'expression multicistronique selon la revendication 2 ou 3, caractérisée en ce qu'elle est fonctionnellement liée à son extrémité 3' avec un terminateur de transcription bactérien.

5. Unité d'expression multicistronique selon les revendications 2 à 4, caractérisée en ce qu'elle est fonctionnellement couplée à un gène "r" comportant son propre promoteur, qui code pour une molécule de répresseur pour le promoteur "p".

6. Unité d'expression multicistronique selon la revendication 5, caractérisée en ce que "p" est le promoteur $\lambda P_R$ ou le promoteur $\lambda P_L$ et "r" est le gène cl857 pour le répresseur $\lambda$ thermosensible.

7. Unité d'expression multicistronique selon les revendications 1 à 6, caractérisée en ce que "TIS" est la séquence TIS B selon SEQ ID n° 15 et/ou TIS E selon SEQ ID n° 14.

8. Unité d'expression multicistronique selon les revendications 1 à 7, caractérisée en ce que les séquences "C" codent pour les divers composants ou les diverses sous-unités de VEGF, du facteur de dispersion (HGF-SF), des membres de la superfamille TGF-$\beta$, de la protéine morphogénique de l'os (BMP), des facteurs de la famille des intégrines/cadhérines, des antigènes d'histocompatibilité, de l'hémoglobine, des récepteurs de lymphocytes T ou de l'hétérodimère AB du facteur de croissance dérivé des plaquettes (PDGF), ou de leurs variants naturels ou synthétiques.

9. Unité d'expression multicistronique selon les revendications 1 à 8, caractérisée en ce que "n" est égal à 2 et les séquences "C" des deux unités répétitives "$(TIS - C)_2$" codent alternativement pour la chaîne A ou la chaîne B de PDGF ou un analogue biologiquement actif ou un fragment de celui-ci, les deux séquences étant représentées simultanément dans l'unité d'expression.

10. Unité d'expression multicistronique selon la revendication 2, caractérisée en ce que l'une des séquences "C" est la séquence de PDGF-$A_K$ (SEQ ID n° 12) ou la séquence de PDGF-$A_L$, qui code pour la chaîne A de PDGF mature.

11. Unité d'expression multicistronique selon la revendication 9 ou 10, caractérisée en ce que l'autre séquence "C" est la séquence complète de PDGF-B (SEQ ID n° 10) qui code pour la chaîne B de PDGF mature (SEQ ID n° 11) ou le gène v-sis du virus du sarcome simien, ou des variantes de ces séquences.

12. Vecteur pour utilisation dans le procédé selon la revendication 1, qui contient une unité d'expression multicistronique selon les revendications 2 à 11.

13. Cellule hôte pour utilisation dans le procédé selon la revendication 1, qui est une cellule bactérienne transformée par un vecteur selon la revendication 12.

14. Cellule hôte selon la revendication 13, caractérisée en ce qu'elle est une cellule hôte de E. *coli.*

15. Unité d'expression selon la revendication 2, caractérisée en ce qu'il s'agit d'une unité d'expression pour l'expression directe et simultanée de séquences d'ADN codant pour PDGF-A et PDGF-B dans une cellule bactérienne, qui présente la formule générale

$$p - TIS_1 - C_1 - TIS_2 - C_2 - t - r$$

dans laquelle

| | |
|---|---|
| "p" | est le promoteur $\lambda P_R$, |
| "$C_1$" et "$C_2$" | contiennent des séquences alternatives qui codent pour PDGF-A ou PDGF-B et les deux gènes sont contenus simultanément dans l'unité d'expression, |
| $TIS_1$ et $TIS_2$ | sont chacune la séquence TIS B (SEQ ID n° 15) ou TIS E (SEQ ID n° 14) et peuvent être identiques ou différentes l'une de l'autre, |
| "t" | est le terminateur du gène de bactériophage fd et |
| "r" | est le gène cI857 du répresseur $\lambda$ thermosensible. |

16. Unité d'expression selon la revendication 15, caractérisée en ce que $C_1$ et $C_2$ contiennent alternativement la séquence de PDGF-A selon SEQ ID n° 12 ou la séquence de PDGF-B selon SEQ ID n° 10 et les deux gènes sont contenus simultanément dans l'unité d'expression.

17. Unité d'expression selon la revendication 16, caractérisée en ce que "$TIS_1$" est la séquence TIS B selon SEQ ID n° 15, "$TIS_2$" est la séquence TIS E selon SEQ ID n° 14, "$C_1$" est la séquence de PDGF-B selon SEQ ID n° 10 et "$C_2$" est la séquence de PDGF-A selon SEQ ID n° 12.

18. Unité d'expression selon la revendication 16, caractérisée en ce que "$TIS_1$" et "$TIS_2$" sont les séquences TIS B selon SEQ ID n° 15, "$C_1$" est la séquence de PDGF-B selon SEQ ID n° 10 et "$C_2$" est la séquence de PDGF-A selon SEQ ID n° 12.

19. Unité d'expression selon la revendication 16, caractérisée en ce que "$TIS_1$" et "$TIS_2$" sont les séquences TIS B selon SEQ ID n° 15, "$C_1$" est la séquence de PDGF-A selon SEQ ID n° 12 et "$C_2$" est la séquence de PDGF-B selon SEQ ID n° 10.

20. Vecteur recombinant pour l'expression dans des cellules bactériennes, qui contient une unité d'expression bicistronique selon les revendications 15 à 19.

21. Cellule hôte, qui est une cellule bactérienne transformée par un vecteur selon la revendication 20.

22. Cellule hôte selon la revendication 21, caractérisée en ce qu'elle est une cellule de *E. coli.*

23. Cellule hôte selon la revendication 22, caractérisée en ce qu'elle est une cellule de *E. coli* qui dérive des clones TG2/pBS/PDGF-BA2 (numéro de dépôt DSM 8336), TG2/pBS/PDGF-BA4 (numéro de dépôt DSM 8338) ou TG2/pBS/PDGF-AB4 (numéro de dépôt DSM 8337).

24. Procédé pour la production par recombinaison de PDGF-AB dans des cellules bactériennes, caractérisé en ce que l'on cultive dans un milieu approprié des cellules hôtes selon les revendications 21 à 23, ensuite on sépare les cellules du milieu, et les composants de protéine PDGF-A et PDGF-B sont isolés à partir du cytoplasme des cellules, renaturés et reconstitués en PDGF-AB fonctionnel.

25. Procédé pour la préparation de compositions pharmaceutiques ou cosmétiques contenant des protéines multimères, caractérisé en ce que l'on prépare les protéines multimères conformément aux étapes a) à c) du procédé

EP 0 701 616 B1

selon la revendication 1 et ensuite, dans l'étape d), les protéines multimères sont formulées, conjointement avec des adjuvants et véhicules usuels, en compositions pharmaceutiques ou cosmétiques.

26. Procédé pour la préparation de compositions pharmaceutiques ou cosmétiques contenant du PDGF-AB, caractérisé en ce que l'on prépare du PDGF-AB conformément au procédé selon la revendication 24 et ensuite il est formulé, conjointement avec des adjuvants et véhicules usuels, en compositions pharmaceutiques ou cosmétiques.

27. Procédé selon la revendication 25 ou 26, caractérisé en ce que la composition pharmaceutique est une pommade, une composition à pulvériser en aérosol, un gel, un pansement, un emplâtre ou une compresse.

# FIGUR 1

# FIGUR 2-1

FIGUR 2-2

FIGUR 3

PDGF-A
PDGF-B

1 2 3 4 5 6 7 8 9

**FIGUR 4**

PDGF-A
PDGF-B

1  2  3  4  5  6  7  8  9  10 11 12 13 14

# FIGUR 5

PDGF-Dimere

PDGF-A
PDGF-B

1    2  3  4  5  6    7  8

# FIGUR 6

PDGF—AB — ELISA

EP 0 701 616 B1

# FIGUR 7

PDGF–AB – ELISA

EP 0 701 616 B1

SEQUENZPROTOKOLL

(1) ALLGEMEINE INFORMATION:

(i) ANMELDER:

(A) NAME: Beiersdorf AG
(B) STRASSE: Unnastr. 48
(C) ORT: Hamburg 20
(E) LAND: Bundesrepublik Deutschland
(F) POSTLEITZAHL: 2000 (20253)

(A) NAME: GBF - Gesellschaft für Biotechnologische Forschung mbH
(B) STRASSE: Mascheroder Weg 1
(C) ORT: Braunschweig
(E) LAND: Bundesrepublik Deutschland
(F) POSTLEITZAHL: 3300

(ii) ANMELDETITEL: Multicistronische Expression rekombinanter Gene in Bakterienzellen.

(iii) ANZAHL DER SEQUENZEN: 18

(iv) COMPUTER-LESBARE FORM:

(A) DATENTRÄGER: Floppy disk
(B) COMPUTER: IBM PC compatible
(C) BETRIEBSSYSTEM: PC-DOS/MS-DOS

(D) SOFTWARE: PatentIn Release #1.0, Version #1.25 (EPA)

(2) INFORMATION ZU SEQ ID NO: 1:

(i) SEQUENZ CHARAKTERISTIKA:

(A) LÄNGE: 919 Basenpaare
(B) ART: Nukleinsäure
(C) STRANGFORM: Einzel
(D) TOPOLOGIE: linear

(ii) ART DES MOLEKÜLS: cDNA

(B) BEMERKUNG: humane, verkürzte PDGF-B Vorläufersequenz, flankiert von 5'-BamHI und 3'-Ncol Restriktionsschnittstellen

(vi) URSPRÜNLICHE HERKUNFT:

(A) ORGANISMUS: Homo sapiens

(vii) UNMITTELBARE HERKUNFT:

(B) CLON: pMVW-2 (Weich et al., 1986)

(ix) MERKMALE:

(A) NAME/SCHLÜSSEL: misc_feature
(B) LAGE: 1..919
(D) SONSTIGE ANGABEN: /function= "BamHI/Ncol-Fragment aus pMVW-2"

(ix) MERKMALE:

(A) NAME/SCHLÜSSEL: CDS
(B) LAGE: 203..850
(D) SONSTIGE ANGABEN: /product= "N-terminal verkürzte PDGF-B vorläufersequenz"

(ix) MERKMALE:

(A) NAME/SCHLÜSSEL: mat_peptide
(B) LAGE: 371..697
(D) SONSTIGE ANGABEN: /product= "mature PDGF-B Kette"

(x) VERÖFFENTLICHUNGSINFORMATION:

(A) AUTOREN: Weich, H. A.
          Sebald, W.
          Schairer, H. U.
          Hoppe, U.
(C) ZEITSCHRIFT: FEBS Lett.
(D) BAND: 198
(F) SEITEN: 344-348
(G) DATUM: 1986

(xi) SEQUENZBESCHREIBUNG: SEQ ID NO: 1:

GGATCCACAG GACGGGTGTG GTCGCCATGA TCGCGTAGTC GATAGTGGCT CCAAGTAGCG 60

AAGCGCCAGG ACTGGGCGGC GGCCAAAGCG GTCGGACAGT GCTCCGAGAA CGGGTGCGCA 120

TAGAAATTGC ATCAACGCAT ATAGCGCTAG CAGCACGCCA TAGTGACTGG CGATGCTGTC 180

```
GGAATGGACG ATCCCCCCCC CC CCC GAG GAG CTT TAT GAG ATG CTG AGT GAC    232
                         Pro Glu Glu Leu Tyr Glu Met Leu Ser Asp
                         -56 -55                     -50
```

```
CAC TCG ATC CGC TCC TTT GAT GAT CTC CAA CGC CTG CTG CAC GGA GAC    280
His Ser Ile Arg Ser Phe Asp Asp Leu Gln Arg Leu Leu His Gly Asp
    -45                 -40                 -35
```

```
CCC GGA GAG GAA GAT GGG GCC GAG TTG GAC CTG AAC ATG ACC CGC TCC    328
Pro Gly Glu Glu Asp Gly Ala Glu Leu Asp Leu Asn Met Thr Arg Ser
-30             -25                 -20                 -15
```

```
CAC TCT GGA GGC GAG CTG GAG AGC TTG GCT CGT GGA AGA AGG AGC CTG    376
His Ser Gly Gly Glu Leu Glu Ser Leu Ala Arg Gly Arg Arg Ser Leu
        -10                 -5                      1
```

```
GGT TCC CTG ACC ATT GCT GAG CCG GCC ATG ATC GCC GAG TGC AAG ACG    424
Gly Ser Leu Thr Ile Ala Glu Pro Ala Met Ile Ala Glu Cys Lys Thr
        5               10                  15
```

```
CGC ACC GAG GTG TTC GAG ATC TCC CGG CGC CTC ATA GAC CGC ACC AAC    472
Arg Thr Glu Val Phe Glu Ile Ser Arg Arg Leu Ile Asp Arg Thr Asn
    20              25                  30
```

```
GCC AAC TTC CTG GTG TGG CCG CCC TGT GTG GAG GTG CAG CGC TGC TCC    520
Ala Asn Phe Leu Val Trp Pro Pro Cys Val Glu Val Gln Arg Cys Ser
35              40                  45                  50
```

```
GGC TGC TGC AAC AAC CGC AAC GTG CAG TGC CGC CCC ACC CAG GTG CAG    568
Gly Cys Cys Asn Asn Arg Asn Val Gln Cys Arg Pro Thr Gln Val Gln
                55                  60                  65
```

```
CTG CGA CCT GTC CAG GTG AGA AAG ATC GAG ATT GTG CGG AAG AAG CCA    616
Leu Arg Pro Val Gln Val Arg Lys Ile Glu Ile Val Arg Lys Lys Pro
            70                  75                  80
```

```
ATC TTT AAG AAG GCC ACG GTG ACG CTG GAA GAC CAC CTG GCA TGC AAG    664
Ile Phe Lys Lys Ala Thr Val Thr Leu Glu Asp His Leu Ala Cys Lys
        85                  90                  95
```

```
TGT GAG ACA GTG GCA GCT GCA CGG CCT GTG ACC CGA AGC CCG GGG GGT    712
Cys Glu Thr Val Ala Ala Ala Arg Pro Val Thr Arg Ser Pro Gly Gly
    100             105                 110
```

```
TCC CAG GAG CAG CGA GCC AAA ACG CCC CAA ACT CGG GTG ACC ATT CGG    760
Ser Gln Glu Gln Arg Ala Lys Thr Pro Gln Thr Arg Val Thr Ile Arg
115             120                 125                 130
```

```
ACG GTG CGA GTC CGC CGG CCC CCC AAG GGC AAG CAC CGG AAA TTC AAG    808
Thr Val Arg Val Arg Arg Pro Pro Lys Gly Lys His Arg Lys Phe Lys
            135                 140                 145
```

```
CAC ACG CAT GAC AAG ACG GCA CTG AAG GAG ACC CTT GGA GCC    850
His Thr His Asp Lys Thr Ala Leu Lys Glu Thr Leu Gly Ala
            150             155                 160
```

TAGGGGCATC GGCAGGAGAG TGTGTGGGCA GGGTTATTTA ATATGGTATT TGCTGTATTG 910

CCCCCATGG 919

(2) INFORMATION ZU SEQ ID NO: 2:

(i) SEQUENZ CHARAKTERISTIKA:

(A) LÄNGE: 216 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 2:

```
Pro Glu Glu Leu Tyr Glu Met Leu Ser Asp His Ser Ile Arg Ser Phe
-56 -55                -50                -45

Asp Asp Leu Gln Arg Leu Leu His Gly Asp Pro Gly Glu Glu Asp Gly
-40                -35                -30                    -25

Ala Glu Leu Asp Leu Asn Met Thr Arg Ser His Ser Gly Gly Glu Leu
             -20                -15                -10

Glu Ser Leu Ala Arg Gly Arg Arg Ser Leu Gly Ser Leu Thr Ile Ala
             -5                1                5

Glu Pro Ala Met Ile Ala Glu Cys Lys Thr Arg Thr Glu Val Phe Glu
         10                15                20

Ile Ser Arg Arg Leu Ile Asp Arg Thr Asn Ala Asn Phe Leu Val Trp
 25                30                35                40

Pro Pro Cys Val Glu Val Gln Arg Cys Ser Gly Cys Cys Asn Asn Arg
             45                50                55

Asn Val Gln Cys Arg Pro Thr Gln Val Gln Leu Arg Pro Val Gln Val
             60                65                70

Arg Lys Ile Glu Ile Val Arg Lys Lys Pro Ile Phe Lys Lys Ala Thr
         75                80                85

Val Thr Leu Glu Asp His Leu Ala Cys Lys Cys Glu Thr Val Ala Ala
     90                95                100

Ala Arg Pro Val Thr Arg Ser Pro Gly Gly Ser Gln Glu Gln Arg Ala
105                110                115                    120

Lys Thr Pro Gln Thr Arg Val Thr Ile Arg Thr Val Arg Val Arg Arg
             125                130                135

Pro Pro Lys Gly Lys His Arg Lys Phe Lys His Thr His Asp Lys Thr
         140                145                150

Ala Leu Lys Glu Thr Leu Gly Ala
         155                160
```

(2) INFORMATION ZU SEQ ID NO: 3:

(i) SEQUENZ CHARAKTERISTIKA:

(A) LÄNGE: 748 Basenpaare

(B) ART: Nukleinsäure
(C) STRANGFORM: Einzel
(D) TOPOLOGIE: linear

(ii) ART DES MOLEKÜLS: cDNA

(B) BEMERKUNG: humanes PDGF-A Gen (kurze Spliceform, Hoppe et al., 1987) aus pODA, flankiert von 5'-EcoRI und 3'-HindIII Restriktionsschnittstellen

(vi) URSPRÜNLICHE HERKUNFT:

(A) ORGANISMUS: Homo sapiens

(vii) UNMITTELBARE HERKUNFT:

(B) CLON: pODA (Eichner et al., 1989)

(ix) MERKMALE:

(A) NAME/SCHLÜSSEL: CDS
(B) LAGE: 95..682
(D) SONSTIGE ANGABEN: /product= "PDGF-A Vorläufersequenz (kurze Spliceform)"

(ix) MERKMALE:

(A) NAME/SCHLÜSSEL: mat_peptide
(B) LAGE: 353..682
(D) SONSTIGE ANGABEN: /product= "mature PDGF-A Kette"

(x) VERÖFFENTLICHUNGSINFORMATION:

(A) AUTOREN: Eichner, W.
       Jaeger, V.
       Herbst, D.
       Hauser, H.
       Hoppe, J.
(C) ZEITSCHRIFT: Eur. J. Biochem.
(D) BAND: 185
(F) SEITEN: 135-140
(G) DATUM: 1989

(x) VERÖFFENTLICHUNGSINFORMATION:

(A) AUTOREN: Hoppe, J.
       Schumacher, L.
       Eichner, W.
       Weich, H. A.
(C) ZEITSCHRIFT: FEBS Lett.
(D) BAND: 223
(F) SEITEN: 243-246
(G) DATUM: 1987

(xi) SEQUENZBESCHREIBUNG: SEQ ID NO: 3

```
GAATTCCCAC TGAATTTCGC CGCCACAGGA GACCGGCTGG AGCGCCCGCC CCGCGCCTCG                60

CCTCTCCTCC GAGCAGCCAG CGCCTCGGGA CGCG ATG AGG ACC TTG GCT TGC                   112
                                       Met Arg Thr Leu Ala Cys
                                       -86 -85

CTG CTG CTC CTC GGC TGC GGA TAC CTC GCC CAT GTT CTG GCC GAG GAA                  160
Leu Leu Leu Leu Gly Cys Gly Tyr Leu Ala His Val Leu Ala Glu Glu
-80             -75                 -70                     -65

GCC GAG ATC CCC CGC GAG GTG ATC GAG AGG CTG GCC CGC AGT CAG ATC                  208
Ala Glu Ile Pro Arg Glu Val Ile Glu Arg Leu Ala Arg Ser Gln Ile
            -60                 -55                 -50

CAC AGC ATC CGG GAC CTC CAG CGA CTC CTG GAG ATA GAC TCC GTA GGG                  256
His Ser Ile Arg Asp Leu Gln Arg Leu Leu Glu Ile Asp Ser Val Gly
            -45                 -40                 -35

AGT GAG GAT TCT TTG GAC ACC AGC CTG AGA GCT CAC GGG GTC CAC GCC                  304
Ser Glu Asp Ser Leu Asp Thr Ser Leu Arg Ala His Gly Val His Ala
        -30                 -25                 -20

ACT AAG CAT GTG CCC GAG AAG CGG CCC CTG CCC ATT CGG AGG AAG AGA                  352
Thr Lys His Val Pro Glu Lys Arg Pro Leu Pro Ile Arg Arg Lys Arg
        -15                 -10                 -5

AGC ATC GAG GAA GCT GTC CCC GCT GTC TGC AAG ACC AGG ACG GTC ATT                  400
Ser Ile Glu Glu Ala Val Pro Ala Val Cys Lys Thr Arg Thr Val Ile
1                   5                   10                  15

TAC GAG ATT CCT CGG AGT CAG GTC GAC CCC ACG TCC GCC AAC TTC CTG                  448
Tyr Glu Ile Pro Arg Ser Gln Val Asp Pro Thr Ser Ala Asn Phe Leu
            20                  25                  30

ATC TGG CCC CCG TGC GTG GAG GTG AAA CGC TGC ACC GGC TGC TGC AAC                  496
Ile Trp Pro Pro Cys Val Glu Val Lys Arg Cys Thr Gly Cys Cys Asn
            35                  40                  45

ACG AGC AGT GTC AAG TGC CAG CCC TCC CGC GTC CAC CAC CGC AGC GTC                  544
Thr Ser Ser Val Lys Cys Gln Pro Ser Arg Val His His Arg Ser Val
            50                  55                  60

AAG GTG GCC AAG GTG GAA TAC GTC AGG AAG AAG CCA AAA TTA AAA GAA                  592
Lys Val Ala Lys Val Glu Tyr Val Arg Lys Lys Pro Lys Leu Lys Glu
65                  70                  75                  80

GTC CAG GTG AGG TTA GAG GAG CAT TTG GAG TGC GCC TGC GCG ACC ACA                  640
Val Gln Val Arg Leu Glu Glu His Leu Glu Cys Ala Cys Ala Thr Thr
            85                  90                  95

AGC CTG AAT CCG GAT TAT CGG GAA GAG GAC ACG GAT GTG AGG                          682
Ser Leu Asn Pro Asp Tyr Arg Glu Glu Asp Thr Asp Val Arg
            100                 105                 110

TGAGGATGAG CCGCAGCCCT TTCCTGGGAC ATGGATGTGG GGATCCGTCG ACCTGCAGCC               742

AAGCTT                                                                          748
```

(2) INFORMATION ZU SEQ ID NO: 4:

    (i) SEQUENZ CHARAKTERISTIKA:

        (A) LÄNGE: 196 Aminosäuren
        (B) ART: Aminosäure
        (D) TOPOLOGIE: linear

(ii) ART DES MOLEKÜLS: Protein

(xi) SEQUENZBESCHREIBUNG: SEQ ID NO: 4:

```
Met Arg Thr Leu Ala Cys Leu Leu Leu Leu Gly Cys Gly Tyr Leu Ala
-86 -85                -80                -75

His Val Leu Ala Glu Glu Ala Glu Ile Pro Arg Glu Val Ile Glu Arg
-70            -65            -60                      -55

Leu Ala Arg Ser Gln Ile His Ser Ile Arg Asp Leu Gln Arg Leu Leu
            -50            -45                -40

Glu Ile Asp Ser Val Gly Ser Glu Asp Ser Leu Asp Thr Ser Leu Arg
            -35            -30                -25

Ala His Gly Val His Ala Thr Lys His Val Pro Glu Lys Arg Pro Leu
        -20            -15                -10

Pro Ile Arg Arg Lys Arg Ser Ile Glu Glu Ala Val Pro Ala Val Cys
    -5                1                5                      10

Lys Thr Arg Thr Val Ile Tyr Glu Ile Pro Arg Ser Gln Val Asp Pro
                15                20                      25

Thr Ser Ala Asn Phe Leu Ile Trp Pro Pro Cys Val Glu Val Lys Arg
                30                35                40

Cys Thr Gly Cys Cys Asn Thr Ser Ser Val Lys Cys Gln Pro Ser Arg
            45                50                55

Val His His Arg Ser Val Lys Val Ala Lys Val Glu Tyr Val Arg Lys
        60                65                70

Lys Pro Lys Leu Lys Glu Val Gln Val Arg Leu Glu Glu His Leu Glu
75                80                85                      90

Cys Ala Cys Ala Thr Thr Ser Leu Asn Pro Asp Tyr Arg Glu Glu Asp
                95                100              105

Thr Asp Val Arg
            110
```

(2) INFORMATION ZU SEQ ID NO: 5:

(i) SEQUENZ CHARAKTERISTIKA:

(A) LÄNGE: 19 Basen
(B) ART: Nukleinsäure
(C) STRANGFORM: Einzel
(D) TOPOLOGIE: linear

(ii) ART DES MOLEKÜLS: synthetische DNA

(A) BEZEICHNUNG: NCCLSA1
(B) BEMERKUNG: synthetischer Linker zur Umklonierung des PDGF-B Gens aus pMVW-2 in den Bakteriophagen M13mp19

(xi) SEQUENZBESCHREIBUNG: SEQ ID NO: 5:

CATGGCCCAA TCGATCCCG                                                    19

(2) INFORMATION ZU SEQ ID NO: 6:

(i) SEQUENZ CHARAKTERISTIKA:

(A) LÄNGE: 19 Basen
(B) ART: Nukleinsäure
(C) STRANGFORM: Einzel
(D) TOPOLOGIE: linear

(ii) ART DES MOLEKÜLS: synthetische DNA

(A) BEZEICHNUNG: NCCLSA2
(B) BEMERKUNG: synthetischer Linker zur Umklonierung des PDGF-B Gens aus pMVW-2 in den Bakteriophagen M13mp19

(xi) SEQUENZBESCHREIBUNG: SEQ ID NO: 6:

TCGACGGGAT CGATTGGGC                                                    19

(2) INFORMATION ZU SEQ ID NO: 7:

(i) SEQUENZ CHARAKTERISTIKA:

(A) LÄNGE: 33 Basen
(B) ART: Nukleinsäure
(C) STRANGFORM: Einzel
(D) TOPOLOGIE: linear

(ii) ART DES MOLEKÜLS: synthetische DNA

(A) BEZEICHNUNG: PDGBNDE
(B) BEMERKUNG: Mutageneseprimer zur Einführung eines ATG-Codons und einer NdeI-Schnittstelle in den 5'-Bereich der maturen PDGF-B Kette

(xi) SEQUENZBESCHREIBUNG: SEQ ID NO: 7:

GCTTGGCTCG TGGACATATG AGCCTGGGTT CCC                                    33

(2) INFORMATION ZU SEQ ID NO: 8:

(i) SEQUENZ CHARAKTERISTIKA:

(A) LÄNGE: 37 Basen
(B) ART: Nukleinsäure
(C) STRANGFORM: Einzel
(D) TOPOLOGIE: linear

(ii) ART DES MOLEKÜLS: synthetische DNA

(A) BEZEICHNUNG: PDGBTGA

(B) BEMERKUNG: Mutageneseprimer zur Einführung eines Stop-Codons an das 3'-Ende der maturen PDGF-B Kette

(xi) SEQUENZBESCHREIBUNG: SEQ ID NO: 8:

GCACGGCCTG TGACCTGATG ACCGGGGGGT TCCCAGG 37

(2) INFORMATION ZU SEQ ID NO: 9:

(i) SEQUENZ CHARAKTERISTIKA:

(A) LÄNGE: 33 Basen
(B) ART: Nukleinsäure
(C) STRANGFORM: Einzel
(D) TOPOLOGIE: linear

(ii) ART DES MOLEKÜLS: synthetische DNA

(A) BEZEICHNUNG: PDGANDE
(B) BEMERKUNG: Mutageneseprimer zur Einführung einer Ndel-Schnittstelle in den 5'-Bereich der maturen PDGF-A Kette

(xi) SEQUENZBESCHREIBUNG: SEQ ID NO: 9:

GCTTCCTCGA TGCTCATATG CCTCCGAATG GGC 33

(2) INFORMATION ZU SEQ ID NO: 10:

(i) SEQUENZ CHARAKTERISTIKA:

(A) LÄNGE: 369 Basenpaare
(B) ART: Nukleinsäure
(C) STRANGFORM: Einzel
(D) TOPOLOGIE: linear

(ii) ART DES MOLEKÜLS: cDNA

(B) BEMERKUNG: humanes PDGF-B Gen nach Einführung eines ATG-Codons und einer Ndel-Schnittstelle in den 5'-Bereich sowie eines Stop-Codons in den 3'-Bereich der maturen PDGF-B Kette

(vi) URSPRÜNLICHE HERKUNFT:

(A) ORGANISMUS: Homo sapiens

(vii) UNMITTELBARE HERKUNFT:

(B) CLON: M13/PDGF-B

(ix) MERKMALE:

(A) NAME/SCHLÜSSEL: mat_peptide
(B) LAGE: 18..347
(D) SONSTIGE ANGABEN: /product= "mature PDGF-B Kette"

(xi) SEQUENZBESCHREIBUNG: SEQ ID NO: 10:

```
GCTTGGCTCG TGGACAT ATG AGC CTG GGT TCC CTG ACC ATT GCT GAG CCG          50
                    Met Ser Leu Gly Ser Leu Thr Ile Ala Glu Pro
                     1               5                  10

GCC ATG ATC GCC GAG TGC AAG ACG CGC ACC GAG GTG TTC GAG ATC TCC          98
Ala Met Ile Ala Glu Cys Lys Thr Arg Thr Glu Val Phe Glu Ile Ser
             15                  20                  25

CGG CGC CTC ATA GAC CGC ACC AAC GCC AAC TTC CTG GTG TGG CCG CCC         146
Arg Arg Leu Ile Asp Arg Thr Asn Ala Asn Phe Leu Val Trp Pro Pro
             30                  35                  40

TGT GTG GAG GTG CAG CGC TGC TCC GGC TGC TGC AAC AAC CGC AAC GTG         194
Cys Val Glu Val Gln Arg Cys Ser Gly Cys Cys Asn Asn Arg Asn Val
     45                  50                  55

CAG TGC CGC CCC ACC CAG GTG CAG CTG CGA CCT GTC CAG GTG AGA AAG         242
Gln Cys Arg Pro Thr Gln Val Gln Leu Arg Pro Val Gln Val Arg Lys
 60                  65                  70                  75

ATC GAG ATT GTG CGG AAG AAG CCA ATC TTT AAG AAG GCC ACG GTG ACG         290
Ile Glu Ile Val Arg Lys Lys Pro Ile Phe Lys Lys Ala Thr Val Thr
                     80                  85                  90

CTG GAA GAC CAC CTG GCA TGC AAG TGT GAG ACA GTG GCA GCT GCA CGG         338
Leu Glu Asp His Leu Ala Cys Lys Cys Glu Thr Val Ala Ala Ala Arg
                 95                  100                 105

CCT GTG ACC TGATGACCGG GGGGTTCCCA GG                                     369
Pro Val Thr
```

(2) INFORMATION ZU SEQ ID NO: 11:

    (i) SEQUENZ CHARAKTERISTIKA:

        (A) LÄNGE: 110 Aminosäuren
        (B) ART: Aminosäure
        (D) TOPOLOGIE: linear

    (ii) ART DES MOLEKÜLS: Protein

    (xi) SEQUENZBESCHREIBUNG: SEQ ID NO: 11:

```
Met Ser Leu Gly Ser Leu Thr Ile Ala Glu Pro Ala Met Ile Ala Glu
 1               5                  10                  15
Cys Lys Thr Arg Thr Glu Val Phe Glu Ile Ser Arg Arg Leu Ile Asp
            20                  25                  30
Arg Thr Asn Ala Asn Phe Leu Val Trp Pro Pro Cys Val Glu Val Gln
            35                  40                  45
Arg Cys Ser Gly Cys Cys Asn Asn Arg Asn Val Gln Cys Arg Pro Thr
        50                  55                  60
Gln Val Gln Leu Arg Pro Val Gln Val Arg Lys Ile Glu Ile Val Arg
65                  70                  75                  80
Lys Lys Pro Ile Phe Lys Lys Ala Thr Val Thr Leu Glu Asp His Leu
                85                  90                  95
Ala Cys Lys Cys Glu Thr Val Ala Ala Ala Arg Pro Val Thr
                100                 105                 110
```

(2) INFORMATION ZU SEQ ID NO: 12:

    (i) SEQUENZ CHARAKTERISTIKA:

        (A) LÄNGE: 352 Basenpaare
        (B) ART: Nukleinsäure
        (C) STRANGFORM: Einzel
        (D) TOPOLOGIE: linear

    (ii) ART DES MOLEKÜLS: cDNA

        (B) BEMERKUNG: humanes PDGF-A Gen nach Einführung eines ATG-Codons und einer Ndel-Schnittstelle in den 5'-Bereich sowie eines Stop-Codons in den 3'-Bereich der maturen PDGF-A Kette

    (vi) URSPRÜNLICHE HERKUNFT:

        (A) ORGANISMUS: Homo sapiens

    (vii) UNMITTELBARE HERKUNFT:

        (B) CLON: pCYT-A-2

    (ix) MERKMALE:

        (A) NAME/SCHLÜSSEL: mat_peptide
        (B) LAGE: 17..349
        (D) SONSTIGE ANGABEN: /product= "mature PDGF-A Kette (kurze Spliceform)"

    (xi) SEQUENZBESCHREIBUNG: SEQ ID NO: 12:

```
GCCCATTCGG AGGCAT ATG AGC ATC GAG GAA GCT GTC CCC GCT GTC TGC AAG          52
                  Met Ser Ile Glu Glu Ala Val Pro Ala Val Cys Lys
                   1               5                   10

ACC AGG ACG GTC ATT TAC GAG ATT CCT CGG AGT CAG GTC GAC CCC ACG          100
Thr Arg Thr Val Ile Tyr Glu Ile Pro Arg Ser Gln Val Asp Pro Thr
         15                  20                  25

TCC GCC AAC TTC CTG ATC TGG CCC CCG TGC GTG GAG GTG AAA CGC TGC          148
Ser Ala Asn Phe Leu Ile Trp Pro Pro Cys Val Glu Val Lys Arg Cys
         30                  35                  40

ACC GGC TGC TGC AAC ACG AGC AGT GTC AAG TGC CAG CCC TCC CGC GTC          196
Thr Gly Cys Cys Asn Thr Ser Ser Val Lys Cys Gln Pro Ser Arg Val
 45                  50                  55                  60

CAC CAC CGC AGC GTC AAG GTG GCC AAG GTG GAA TAC GTC AGG AAG AAG          244
His His Arg Ser Val Lys Val Ala Lys Val Glu Tyr Val Arg Lys Lys
                 65                  70                  75

CCA AAA TTA AAA GAA GTC CAG GTG AGG TTA GAG GAG CAT TTG GAG TGC          292
Pro Lys Leu Lys Glu Val Gln Val Arg Leu Glu Glu His Leu Glu Cys
                 80                  85                  90

GCC TGC GCG ACC ACA AGC CTG AAT CCG GAT TAT CGG GAA GAG GAC ACG          340
Ala Cys Ala Thr Thr Ser Leu Asn Pro Asp Tyr Arg Glu Glu Asp Thr
         95                  100                 105

GAT GTG AGG TGA                                                          352
Asp Val Arg
        110
```

(2) INFORMATION ZU SEQ ID NO: 13:

    (i) SEQUENZ CHARAKTERISTIKA:

        (A) LÄNGE: 111 Aminosäuren
        (B) ART: Aminosäure
        (D) TOPOLOGIE: linear

    (ii) ART DES MOLEKÜLS: Protein

    (xi) SEQUENZBESCHREIBUNG: SEQ ID NO: 13:

```
Met Ser Ile Glu Glu Ala Val Pro Ala Val Cys Lys Thr Arg Thr Val
 1               5                   10                  15

Ile Tyr Glu Ile Pro Arg Ser Gln Val Asp Pro Thr Ser Ala Asn Phe
             20                  25                  30

Leu Ile Trp Pro Pro Cys Val Glu Val Lys Arg Cys Thr Gly Cys Cys
         35                  40                  45

Asn Thr Ser Ser Val Lys Cys Gln Pro Ser Arg Val His His Arg Ser
         50                  55                  60

Val Lys Val Ala Lys Val Glu Tyr Val Arg Lys Lys Pro Lys Leu Lys
 65                  70                  75                  80
```

```
Glu Val Gln Val Arg Leu Glu Glu His Leu Glu Cys Ala Cys Ala Thr
                85                    90                    95

Thr Ser Leu Asn Pro Asp Tyr Arg Glu Glu Asp Thr Asp Val Arg
                100                   105                   110
```

(2) INFORMATION ZU SEQ ID NO: 14:

    (i) SEQUENZ CHARAKTERISTIKA:

        (A) LÄNGE: 58 Basenpaare
        (B) ART: Nukleinsäure
        (C) STRANGFORM: Einzel
        (D) TOPOLOGIE: linear

    (ii) ART DES MOLEKÜLS: synthetische DNA

        (A) BEZEICHNUNG: TIS E Sequenz
        (B) BEMERKUNG: flankiert von 5' XhoI und 3' NdeI Restriktionsschnittstellen

    (xi) SEQUENZBESCHREIBUNG: SEQ ID NO: 14:

```
CTCGAGTAAT TTACCAACAC TACTACGTTT TAACTGAAAC AAACTGGAGA CTCATATG          58
```

(2) INFORMATION ZU SEQ ID NO: 15:

    (i) SEQUENZ CHARAKTERISTIKA:

        (A) LÄNGE: 35 Basenpaare
        (B) ART: Nukleinsäure
        (C) STRANGFORM: Einzel
        (D) TOPOLOGIE: linear

    (ii) ART DES MOLEKÜLS: synthetische DNA

        (A) BEZEICHNUNG: TIS B Sequenz
        (B) BEMERKUNG: flankiert von 5' SpeI und 3' NdeI Restriktionsschnittstellen

    (xi) SEQUENZBESCHREIBUNG: SEQ ID NO: 15:

```
ACTAGTAAAA AGAAAGGAGG TGATCAAAAC ATATG                                    35
```

(2) INFORMATION ZU SEQ ID NO: 16:

    (i) SEQUENZ CHARAKTERISTIKA:

        (A) LÄNGE: 1552 Basenpaare
        (B) ART: Nukleinsäure
        (C) STRANGFORM: Doppel
        (D) TOPOLOGIE: linear

    (ii) ART DES MOLEKÜLS:

        (A) BEZEICHNUNG: Expressionskassette des pCYTEX P3 Vektors

(B) BEMERKUNG: 5' und 3' flankiert von pUC18 Nukleotidsequenz

(xi) SEQUENZBESCHREIBUNG: SEQ ID NO: 16:

```
CATATAGCGG CCGCAGGTGA TGATTATCAG CCAGCAGAGA ATTAAGGAAA ACAGACAGGT    60

TTATTGAGCG CTTATCTTTC CCTTTATTTT TGCTGCGGTA AGTCGCATAA AAACCATTCT   120

TCATAATTCA ATCCATTTAC TATGTTATGT TCTGAGGGGA GTGAAAATTC CCCTAATTCG   180

ATGAAGATTC TTGCTCAATT GTTATCAGCT ATGCGCCGAC CAGAACACCT TGCCGATCAG   240

CCAAACGTCT CTTCAGGCCA CTGACTAGCG ATAACTTTCC CCACAACGGA ACAACTCTCA   300

TTGCATGGGA TCATTGGGTA CTGTGGGTTT AGTGGTTGTA AAAACACCTG ACCGCTATCC   360

CTGATCAGTT TCTTGAAGGT AAACTCATCA CCCCCAAGTC TGGCTATGCA GAAATCACCT   420

GGCTCAACAG CCTGCTCAGG GTCAACGAGA ATTAACATTC CGTCAGGAAA GCTTGGCTTG   480

GAGCCTGTTG GTGCGGTCAT GGAATTACCT TCAACCTCAA GCCAGAATGC AGAATCACTG   540

GCTTTTTTGG TTGTGCTTAC CCATCTCTCC GCATCACCTT TGGTAAAGGT TCTAAGCTTA   600

GGTGAGAACA TCCCTGCCTG AACATGAGAA AAAACAGGGT ACTCATACTC ACTTCTAAGT   660

GACGGCTGCA TACTAACCGC TTCATACATC TCGTAGATTT CTCTGGCGAT TGAAGGGCTA   720

AATTCTTCAA CGCTAACTTT GAGAATTTTT GTAAGCAATG CGGCGTTATA AGCATTTAAT   780

GCATTGATGC CATTAAATAA AGCACCAACG CCTGACTGCC CCATCCCCAT CTTGTCTGCG   840

ACAGATTCCT GGGATAAGCC AAGTTCATTT TTCTTTTTTT CATAAATTGC TTTAAGGCGA   900

CGTGCGTCCT CAAGCTGCTC TTGTGTTAAT GGTTTCTTTT TTGTGCTCAT ACGTTAAATC   960

TATCACCGCA AGGGATAAAT ATCTAACACC GTGCGTGTTG ACTATGGTAC CCGGGGATCG  1020

ATCCCTCCTT AAATCTATCA CCGCAAGGGA TAAATATCTA ACACCGTGCG TGTTGACTAT  1080

TTTACCTCTG GCGGTGATAA TGGTTGCATA GATCTACTAG TAAAAAGAAA GGAGGTGATC  1140

AAAACATATG TCTAGAGGAT CCGTCGACTC TAGAGGATCT AAAGTTTTGT CGTCTTTCCA  1200

GACGTTAGTA AATGAATTTT CTGTATGAGG TTTTGCTAAA CAACTTTCAA CAGTTTCAGC  1260

GGAGTGAGAA TAGAAAGGAA CAACTAAAGG AATTGCGAAT AATAATTTTT TCACGTTGAA  1320

AATCTCCAAA AAAAAAGGCT CCAAAAGGAG CCTTTAATTG TATCGGTTTA TCAGCTTGCT  1380

TTCGAGGTGA ATTTCTTAAA CAGCTTGATA CCGATAGTTG CGCCGACAAT GACAACAACC  1440

ATCGCCCACG CATAACCGAT ATATTCGGTC GCTGAGGCTT GCAGGGAGTC AAAGGCGCTT  1500

TTGCGGGATC TCGTCGAAGG CGCGGGGGCG CGGACGCCGC CGGGTTCCCC TG          1552
```